# EUROPEAN PATENT APPLICATION

(11) **EP 4 737 475 A1**
(43) Date of publication of application: **06.05.2026**
(21) Application number: 24846047.9
(22) Date of filing: 24.07.2024
(51) Int. Cl.: C07K 14/34, C12N 15/01, C12N 15/77, C12P 13/06

(54) **PROTEIN VARIANT AND METHOD FOR PRODUCING L-ISOLEUCINE BY USING SAME**

(30) Priority: 24.07.2023 KR 20230096087
(71) Applicant: CJ Cheiljedang Corporation, Seoul 04560 (KR)
(72) Inventor: YOON, Jeong-Hye, Seoul 04560 (KR); KIM, Gyuree, Seoul 04560 (KR); JUNG, Moo Young, Seoul 04637 (KR); SHIM, Jihyun, Seoul 04560 (KR); SEO, Chang Il, Seoul 04560 (KR)
(74) Representative: Meissner Bolte Partnerschaft mbB
(86) International application number: PCT/KR2024/010752
(87) International publication number: WO 2025/023745

(57) **Abstract**

The present disclosure relates to: a protein variant; a polynucleotide encoding the protein variant; a microorganism of the genus Corynebacterium, comprising at least one selected from the group consisting of the protein variant and the polynucleotide; and a method for producing L-isoleucine by using the microorganism.

## Description

### [TECHNICAL FIELD]

### Cross-reference to related application(s)

The present disclosure claims the benefit of the priority based on Korean Patent Application No. 10-2023-0096087 filed on July 24, 2023, and the entire contents disclosed in the document of the corresponding Korean patent application are incorporated as a part of the present disclosure.

Throughout the present disclosure, numerous papers and patent documents are referenced and citations thereof are indicated. The disclosures of the cited papers and patent documents are incorporated into the present disclosure in their entirety by reference to more clearly describe the level of the technical field to which the present invention pertains and the contents of the present invention.

The present disclosure relates to a protein variant, a microorganism of the genus of *Corynebacterium* comprising the protein variant, and a method of producing L-isoleucine using the microorganism.

### [BACKGROUND ART]

L-isoleucine (lle, I) is classified as an essential amino acid and is one of the 20 essential amino acids. It belongs to the group of branched-chain amino acids (BCAAs) and is used in the production of various products such as animal feed, food additives, and pharmaceuticals. Because L-isoleucine contributes to energy generation after metabolism, hemoglobin synthesis, blood sugar regulation, and muscle building and maintenance, its applications are expanding not only in infusion solutions, nutritional supplements, and sports nutrition but also in the field of animal feed.

Based on this trend, various attempts have been made to enhance the production capacity of L-isoleucine using microorganisms, including those belonging to the genus Corynebacterium (US6072083A).

Despite these efforts, there remains a continuing need to develop technologies that can further improve the productivity of L-isoleucine.

### [DISCLOSURE]

### [TECHNICAL PROBLEM]

One object of the present disclosure is to provide a protein variant.

Another object of the present disclosure is to provide a polypeptide encoding the protein variant.

Other object of the present disclosure is to provide a microorganism of the genus of *Corynebacterium,* comprising at least one selected from the group consisting of the protein variant and the polynucleotides encoding the same.

Other object of the present disclosure is to provide a method of producing L-isoleucine, comprising culturing the microorganism in a medium.

Other object of the present disclosure is to provide a use for producing L-isoleucine of the microorganism.

### [TECHNICAL SOLUTION]

Description thereof in detail is as follows. On the other hand, each description, aspect and embodiment disclosed in the present disclosure can also be applied to each other description, aspect and embodiment. In other words, all combinations of various elements disclosed in the present disclosure belong to the scope of the present disclosure. In addition, the scope of the present disclosure is not to be considered limited by the specific description described below. Furthermore, those skilled in the art can recognize or confirm numerous equivalents to the specific aspects of the present application disclosed in the present application using only common experiments. In addition, these equivalents are intended to be included in the present application.

One aspect of the present disclosure provides a protein variant comprising an amino acid sequence in which the amino acid corresponding to the 27th position from the N-terminus in the amino acid sequence of SEQ ID NO:116 is substituted with an amino acid different from the original amino acid, an amino acid sequence in which the amino acid corresponding to the 461st position from the N-terminus in the amino acid sequence of SEQ ID NO:117 is substituted with an amino acid different from the original amino acid, or an amino acid sequence in which the amino acid corresponding to the 75th position from the N-terminus in the amino acid sequence of SEQ ID NO:118 is substituted with an amino acid different from the original amino acid.

In the present disclosure, the sequence of SEQ ID NOs: 116 to 118 are shown in table 1 below.

**[Table 1]**

| Protein name (gene ID, gene name) | Sequence (N-terminus → C-terminus) | SEQ ID NO |
|---|---|---|
| flavoprotein disulfide reductase (NCgI0658, IpdA) | MAKRIVIIGGGPAGYEAALAGAKYGAEVTVIEDVGV GGSAVTMDCVPSKSFIAGTGIKTDLRRADDMGLNR GLGKAHLEIDALNIRVKDLAKAQSEDILGQLQRSDV RMINGVGRFDDYNTKQTTHYIKVTHSDGSEETVEC DLVLVATGATPRILKGAEPDGERILTWRQVYDIEELP THLIVVGSGVTGAEFVSAFAELGVKVTMVASRDRIL PHDDADAADVLETVLAERGVSLEKHARVESVTRTE DGGVCVRTADGREIYGSHALMTVGSIPNTADLGLE NIGVELAPSGHIKVDRVSRTNIPGVYAAGDCTDLFPL ASVAAMQGRIAMYHALGEGVSPIRLKTVATAVFTRP EIAAVGITHAQVDSGEVSARVIVLPLATNPRAKMRSL RHGFVKLFCRRNSGLIIGGVVVAPTASELILPIAVAVT NRLTVADLADTFAVYPSLSGSITEAARQLVQHDDLG | 116 |
| ATP synthase F0F1 subunit beta (NCgI1165, atpD) | MTTALEEQNAQQAATAGRVVRVIGAVVDVEFPRGE LPALYNALTVEVTLESVKKTVVLEVAQHLGDNLIRTI AMAPTDGLVRGAAVTDTARPISVPVGDVVKGHVFN ALGDCLDDVSLNNNPEIERWGIHREPPSFDQLEGK TEILETGIKVIDLLTPYVKGGKIGLFGGAGVGKTVLIQ EMITRIAREFSGTSVFAGVGERTREGTDLFLEMEEM GVLQDTALVFGQMDEPPGVRMRVALSGLTMAEYF RDVQNQDVLLFIDNIFRFTQAGSEVSTLLGRMPSAV GYQPTLADEMGVLQERITSTKGRSITSLQAVYVPAD DYTDPAPATTFAHLDATTELDRSIASKGIYPAVNPLT STSRILEPAIVGERHYEVSQRVIGILQKNKELQDIIAIL GMDELSEEDKITVARARRIERFLGQNFFVAEKFTGL PGSYVPLTDTVDAFERICNGDFDHYPDQAFNGLGG LDDVEAAYKKLTGK | 117 |
| transcriptional regulator of sugar metabolism (NCgI1859, fruR) | MVSQTERQHAIASLLAPTGAVSVGDLAEHFHVTTET VRRDLRIMESLGLLQRVHGGAISPEPMGTSPPRLKP ALGKGMPPEPRVLELAETAVSLITPLARSIFLDSGLA CTAIATVLGDPPEDARWTVVTSSPGAVIALSATDAT STVVLHGQVHGNCSSIIGSTAVDMISQLRADIAFVEV DAIQSDTSLCTFFPETIPIKQAMIKNAAFTVAVLSPRS PQDQELQLLKHPFSTLADFDALVTDDHTLDFPVLPD HNFQVVTP | 118 |

In one embodiment, the afore-mentioned protein variant of the present disclosure may be essentially consisting of an amino acid sequence in which the amino acid corresponding to the 27th position from the N-terminus in the amino acid sequence of SEQ ID NO:116 is substituted with an amino acid different from the original amino acid, an amino acid sequence in which the amino acid corresponding to the 461st position from the N-terminus in the amino acid sequence of SEQ ID NO:117 is substituted with an amino acid different from the original amino acid, or an amino acid sequence in which the amino acid corresponding to the 75th position from the N-terminus in the amino acid sequence of SEQ ID NO:118 is substituted with an amino acid different from the original amino acid. In another embodiment, the afore-mentioned protein variant of the present disclosure may be consisting of an amino acid sequence in which the amino acid corresponding to the 27th position from the N-terminus in the amino acid sequence of SEQ ID NO:116 is substituted with an amino acid different from the original amino acid, an amino acid sequence in which the amino acid corresponding to the 461st position from the N-terminus in the amino acid sequence of SEQ ID NO:117 is substituted with an amino acid different from the original amino acid, or an amino acid sequence in which the amino acid corresponding to the 75th position from the N-terminus in the amino acid sequence of SEQ ID NO:118 is substituted with an amino acid different from the original amino acid.

In the present disclosure, the amino acid sequence of SEQ ID NO: 116 may be the amino acid sequence of IpdA (flavoprotein disulfide reductase) protein. In one embodiment, the IpdA protein may be a protein encoded by the NCgI0658 gene.

In the present disclosure, the amino acid sequence of SEQ ID NO: 117 may be the amino acid sequence of AtpD (ATP synthase F0F1 subunit beta) protein. In one embodiment, the AtpD protein may be a protein encoded by the NCgl1165 gene.

In the present disclosure, the amino acid sequence of SEQ ID NO: 118 may be the amino acid sequence of FruR (transcriptional regulator of sugar metabolism) protein. In one embodiment, the FruR protein may be a protein encoded by the NCg11859 gene.

In one embodiment, the amino acid corresponding to the 27th position from the N-terminus in the amino acid sequence of SEQ ID NO: 116 may be glutamic acid (Glu, E), but not limited thereto.

In one embodiment, the amino acid corresponding to the 461st position from the N-terminus in the amino acid sequence of SEQ ID NO: 117 may be aspartic acid (Asp, D), but not limited thereto.

In one embodiment, the amino acid corresponding to the 75th position from the N-terminus in the amino acid sequence of SEQ ID NO: 118 may be glycine (Gly, G), but not limited thereto.

In one embodiment, the amino acid corresponding to the 27th position from the N-terminus in the amino acid sequence of SEQ ID NO: 116 may be substituted with aspartic acid (Asp, D), but not limited thereto.

In one embodiment, the amino acid corresponding to the 461st position from the N-terminus in the amino acid sequence of SEQ ID NO: 117 may be substituted with glutamic acid (Glu, E), but not limited thereto.

In one embodiment, the amino acid corresponding to the 75th position from the N-terminus in the amino acid sequence of SEQ ID NO: 118 may be substituted with valine (Val, V), but not limited thereto.

The protein variant of the present disclosure may have activity to increase L-isoleucine productivity compared to a wild-type protein (polypeptide).

The protein variant of the present disclosure may comprise an amino acid sequence having homology or identity of at least 70%, 75%, 80%, 85%, 90%, 95%, 96%, 97%, 98%, 99%, 99.5%, or 99.7% or more to an amino acid sequence in which the amino acid corresponding to the 27th position from the N-terminus in the amino acid sequence of SEQ ID NO:116 is substituted with an amino acid (for example, aspartic acid) different from the original amino acid, an amino acid sequence in which the amino acid corresponding to the 461st position from the N-terminus in the amino acid sequence of SEQ ID NO:117 is substituted with an amino acid (for example, glutamic acid) different from the original amino acid, or an amino acid sequence in which the amino acid corresponding to the 75th position from the N-terminus in the amino acid sequence of SEQ ID NO:118 is substituted with an amino acid (for example, valine) different from the original amino acid. In addition, it is obvious that even a variant having an amino acid sequence in which some sequences are deleted, modified, substituted, conservatively substituted, or added is included in the scope of the present disclosure, as long as it is an amino acid sequence which has such homology or identity and shows efficacy (efficacy of increasing L-isoleucine production) corresponding to the variant of the present disclosure.

For example, they are cases of having addition or deletion of a sequence which does not change the function of the variant of the present disclosure, naturally occurring mutation, silent mutation, or conservative substitution at the N-terminus, C-terminus and/or inside the amino acid sequences.

The "conservative substitution" refers to substituting one amino acid with an amino acid different from the original amino acid having similar structural and/or chemical characteristics. This amino acid substitution may occur generally based on the similarity in polarity, charge, solubility, hydrophobicity, hydrophilicity, and/or amphipathic nature of a residue. Commonly, the conservative substitution may hardly affect or not affect activity of a protein or polypeptide.

In the present disclosure, the term, "variant" refers to a polypeptide which is different from the amino acid sequence before mutation of the variant, as at least one amino acid is under conservative substitution and/or modification, but functions or properties are maintained. Such a variant may be commonly identified by modifying at least one amino acid among the amino acid sequence of the polypeptide, and evaluating the properties of the modified polypeptide. In other words, the ability of the variant may increase, or be not changed, or decrease, compared the polypeptide before mutation. In addition, some variants may include variants in which at least one part such as an N-terminal leader sequence or transmembrane domain is removed. Other variants may include variants in which some parts from the N- and/or C-terminus of a mature protein are removed. The term "variant" may be interchangeably used with terms such as mutant, modification, modified polypeptide, mutated protein, mutation and variant and the like (modification, modified polypeptide, modified protein, mutant, mutein, divergent, etc. as English expressions), and even if it is a term used as the mutated meaning, it is not limited thereto.

In addition, the variant may comprise deletion or addition of amino acids having minimal effects on the properties and secondary structure of the polypeptide. For example, a signal (or leader) sequence related to co-translational or post-translational translocation of proteins may be conjugated at the N-terminus. Furthermore, the variant may be conjugated with another sequence or a linker to be identified, purified, or synthesized.

In one embodiment, the afore-mentioned protein variant of the present disclosure may have homology or identity of 90%, 91%, 93%, 94%, 95%, 96%, 97%, 98%, or 99% or more, less than 100% to the amino acid sequence of SEQ ID NO: 116, may have homology or identity of 90%, 91%, 93%, 94%, 95%, 96%, 97%, 98%, or 99% or more, less than 100% to the amino acid sequence of SEQ ID NO: 117, or may have homology or identity of 90%, 91%, 93%, 94%, 95%, 96%, 97%, 98%, or 99% or more, less than 100% to the amino acid sequence of SEQ ID NO: 118, but be not limited thereto. In other words, the afore-mentioned protein variant of the present disclosure may have the amino acid sequence identity of 90%, 91%, 93%, 94%, 95%, 96%, 97%, 98%, or 99% or more, less than 100%, respectively, to the amino acid sequence of SEQ ID NO: 116, SEQ ID NO: 117 or SEQ ID NO: 118.

Another aspect of the present disclosure provides a protein variant, comprising at least one amino acid sequence selected from the group consisting of amino acid sequences of SEQ ID NOs: 1 to 19. In one embodiment, the protein variant may be a protein variant comprising the amino acid sequence of SEQ ID NO: 2, SEQ ID NO: 8, or SEQ ID NO: 14. In other embodiment, the protein variant may be essentially consisting of the amino acid sequence of SEQ ID NO: 2, SEQ ID NO: 8, or SEQ ID NO: 14. In other embodiment, the protein variant may be consisting of the amino acid sequence of SEQ ID NO: 2, SEQ ID NO: 8, or SEQ ID NO: 14.

In the present disclosure, the amino acid sequence of SEQ ID NO: 2 may be the sequence of a protein variant in which the 27th amino acid of the protein (flavoprotein disulfide reductase) encoded by the NCgI0658 gene is substituted from glutamic acid (Glu, E) to aspartic acid (Asp, D).

In the present disclosure, the amino acid sequence of SEQ ID NO:8 may be the sequence of a protein variant in which the 461st amino acid of the protein (ATP synthase F0F1 subunit beta) encoded by the NCgl1165 gene is substituted from aspartic acid (Asp, D) to glutamic acid (Glu, E).

In the present disclosure, the amino acid sequence of SEQ ID NO:14 may be the sequence of a protein variant in which the 75th amino acid of the protein (transcriptional regulator of sugar metabolism) encoded by the NCgl1859 gene is substituted from glycine (Gly, G) to valine (Val, V).

The protein variant of the present disclosure may comprise any one amino acid sequence (for example, the amino acid sequence of SEQ ID NO: 2, SEQ ID NO: 8, or SEQ ID NO: 14) selected from the group consisting of amino acid sequences of SEQ ID NOs: 1 to 19, or comprise an amino acid sequence having homology or identity of at least 70%, 75%, 80%, 85%, 90%, 95%, 96%, 97%, 98%, 99%, 99.5%, 99.7% or 99.9% or more to said amino acid sequence. In addition, it is obvious that even a variant having an amino acid sequence in which some sequences are deleted, modified, substituted, conservatively substituted, or added is included in the scope of the present disclosure, as long as it is an amino acid sequence which has such homology or identity and shows efficacy (efficacy of increasing L-isoleucine production) corresponding to the variant of the present disclosure.

For example, they are cases of having addition or deletion of a sequence which does not change the function of the variant of the present disclosure, naturally occurring mutation, silent mutation, or conservative substitution at the N-terminus, C-terminus and/or inside the amino acid sequences.

In the protein variant, the conservative substitution, variant, and the like are as described above.

In the present disclosure, the term, 'homology' or 'identity' means the degree of similarity between two given amino acid sequences or nucleotide sequences, and may be expressed as a percentage. The terms, homology and identity may often be used interchangeably.

The sequence homology or identity of a conserved polynucleotide or polypeptide may be determined by a standard alignment algorithm, and a default gap penalty established by the program used may be used together. Substantially, a homologous or identical sequence may be generally hybridized with all or a part of a sequence under medium or high stringent conditions. It is obvious that hybridization also includes hybridization of a polynucleotide with a polynucleotide containing common codons or codons considering codon degeneracy.

Whether any two polynucleotide or polypeptide sequences have homology, similarity, or identity, may be determined using a known computer algorithm such as "FASTA" program using a default parameter such as that in for example, Pearson et al (1988) [Proc. Natl. Acad. Sci. USA 85]: 2444. Otherwise, it may be determined using Needleman-Wunsch algorithm (Needleman and Wunsch, 1970, J. Mol. Biol. 48: 443-453), as performed in Needleman program of EMBOSS package (EMBOSS: The European Molecular Biology Open Software Suite, Rice et al., 2000, Trends Genet. 16: 276-277) (version 5.0.0 or later version) (GCG program package (including Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego, 1994, and [CARILLO ETA/.](1988) SIAM J Applied Math 48: 1073). For example, the homology, similarity, or identity may be determined using BLAST or ClustalW of National Database Center of Biotechnology Information.

The homology, similarity, or identity of the polynucleotide or polypeptide may be determined by compared sequence information using for example, GAP computer program such as Needleman et al. (1970), J Mol Biol. 48:443, for example, known in Smith and Waterman, Adv. Appl. Math (1981) 2:482. In summary, the GAP program may be defined as a value of dividing a total number of symbols in the shorter of two sequences by the number of similar aligned symbols (i.e., nucleotides or amino acids). The default parameters for the GAP program may comprise (1) a binary comparison matrix (containing values of 1 for identity and 0 for non-identity) and a weighted comparison matrix of Gribskov et al (1986) Nucl. Acids Res. 14: 6745, disclosed in Schwartz and Dayhoff, eds., Atlas Of Protein Sequence And Structure, National Biomedical Research Foundation, pp. 353-358 (1979) (or EDNAFULL (EMBOSS version of NCBI NUC4.4) substitution matrix); (2) a 3.0 penalty for each gap and an additional 0.10 penalty for each symbol in each gap (or gap opening penalty 10, gap extension penalty 0.5); and (3) no penalty for end gaps.

As one example of the present disclosure, the variant of the present disclosure may have activity to increase L-isoleucine productivity compared to a wild-type protein (polypeptide).

In the present disclosure, the term, "corresponding to" refers to an amino acid residue at a position listed in a polypeptide, or an amino acid residue similar or identical or homologous to a residue listed in a polypeptide. Confirming an amino acid at a corresponding position may determine a specific amino acid of a sequence referring to a specific sequence. The "corresponding region" used in the present disclosure generally refers to a similar or corresponding position in a related protein or reference protein.

For example, any amino acid sequence may be aligned with an amino acid sequence in which the amino acid corresponding to the 27th position from the N-terminus in the amino acid sequence of SEQ ID NO:116 is substituted with an amino acid (for example, aspartic acid) different from the original amino acid, an amino acid sequence in which the amino acid corresponding to the 461st position from the N-terminus in the amino acid sequence of SEQ ID NO:117 is substituted with an amino acid (for example, glutamic acid) different from the original amino acid, or an amino acid sequence in which the amino acid corresponding to the 75th position from the N-terminus in the amino acid sequence of SEQ ID NO:118 is substituted with an amino acid (for example, valine) different from the original amino acid; or any one amino acid sequence selected from the group consisting of SEQ ID NOs: 1 to 19 (for example, the amino acid sequence of SEQ ID NO: 2, SEQ ID NO: 8 or SEQ ID NO: 14), and based on this, each amino acid residue of the amino acid sequence may be numbered with reference to the number position of the amino acid residue corresponding to an amino acid sequence in which the amino acid corresponding to the 27th position from the N-terminus in the amino acid sequence of SEQ ID NO:116 is substituted with an amino acid (for example, aspartic acid) different from the original amino acid, an amino acid sequence in which the amino acid corresponding to the 461st position from the N-terminus in the amino acid sequence of SEQ ID NO:117 is substituted with an amino acid (for example, glutamic acid) different from the original amino acid, or an amino acid sequence in which the amino acid corresponding to the 75th position from the N-terminus in the amino acid sequence of SEQ ID NO:118 is substituted with an amino acid (for example, valine) different from the original amino acid; or any one amino acid sequence selected from the group consisting of SEQ ID NOs: 1 to 19 (for example, the amino acid sequence of SEQ ID NO: 2, SEQ ID NO: 8 or SEQ ID NO: 14). For example, the sequence alignment algorithm as described in the present disclosure, may confirm a location of amino acids, or a location where modification such as substitution, insertion or deletion or the like occurs, compared to a query sequence (also called "reference sequence").

For this alignment, for example, Needleman-Wunsch algorithm (Needleman and Wunsch, 1970, J. Mol. Biol. 48: 443-453), Needle program of EMBOSS package (EMBOSS: The European Molecular Biology Open Software Suite, Rice et al., 2000), Trends Genet. 16: 276-277) and the like may be used, but not limited thereto, and a sequence alignment program known in the art, pairwise sequence comparison algorithm and the like may be appropriately used.

Other aspect of the present disclosure provides a polynucleotide encoding the protein variant of the present disclosure.

In the present disclosure, the term, "polynucleotide" refers to a DNA or RNA strand of a certain length or more, which is a polymer of nucleotides in which nucleotide monomers are connected in a long chain form by covalent bonds, and more specifically, a polynucleotide fragment encoding the variant.

In one embodiment, the polynucleotide may be a polynucleotide encoding a protein variant comprising an amino acid sequence in which the amino acid corresponding to the 27th position from the N-terminus in the amino acid sequence of SEQ ID NO:116 is substituted with an amino acid (for example, aspartic acid) different from the original amino acid, an amino acid sequence in which the amino acid corresponding to the 461st position from the N-terminus in the amino acid sequence of SEQ ID NO:117 is substituted with an amino acid (for example, glutamic acid) different from the original amino acid, or an amino acid sequence in which the amino acid corresponding to the 75th position from the N-terminus in the amino acid sequence of SEQ ID NO:118 is substituted with an amino acid (for example, valine) different from the original amino acid.

In one embodiment, the polynucleotide may comprise a polynucleotide comprising any one nucleotide sequence selected from the group consisting of nucleotide sequences of SEQ ID NOs: 20 to 38, for example, the nucleotide sequence of SEQ ID NO: 21, SEQ ID NO: 27 or SEQ ID NO:33.

The polynucleotide encoding the protein variant of the present disclosure may have or comprise any one nucleotide sequence selected from the group consisting of nucleotide sequences of a polynucleotide encoding a protein variant comprising an amino acid sequence in which the amino acid corresponding to the 27th position from the N-terminus in the amino acid sequence of SEQ ID NO:116 is substituted with an amino acid (for example, aspartic acid) different from the original amino acid, an amino acid sequence in which the amino acid corresponding to the 461st position from the N-terminus in the amino acid sequence of SEQ ID NO:117 is substituted with an amino acid (for example, glutamic acid) different from the original amino acid, or an amino acid sequence in which the amino acid corresponding to the 75th position from the N-terminus in the amino acid sequence of SEQ ID NO:118 is substituted with an amino acid (for example, valine) different from the original amino acid, or any one nucleotide sequence selected from the group consisting of nucleotide sequences of SEQ ID NOs: 20 to 38, for example, the nucleotide sequence of SEQ ID NO: 21, SEQ ID NO: 27 or SEQ ID NO:33. In another embodiment, the polynucleotide may comprise a nucleotide sequence having homology or identity of at least 70%, 75%, 80%, 85%, 90%, 95%, 96%, 97%, 98%, 99%, 99.5%, 99.7% or 99.9% or more to the nucleotide sequence. In addition, it is obvious that even a polynucleotide having a nucleotide sequence in which some sequences are deleted, modified, substituted, conservatively substituted, or added is included in the scope of the present disclosure, as long as it is a sequence which has such homology or identity and shows efficacy (efficacy of increasing L-isoleucine production) corresponding to the variant of the present disclosure.

In one embodiment, the nucleotide sequence or amino acid sequence provided in the present description may include one modified by common mutagenesis, for example, direct evolution and/or site-directed mutagenesis and the like, within a range of maintaining their original functions or desired functions. In one embodiment, that a polynucleotide or polypeptide "comprises a specific nucleotide or amino acid sequence" may mean that the polynucleotide or polypeptide (i) consists of the specific nucleotide sequence or amino acid sequence, or essentially comprises the same, or (ii) consists of an amino acid sequence having homology of 60% or more, 70% or more, 80% or more, 85% or more, 90% or more, 91% or more, 92% or more, 93% or more, 94% or more, 95% or more, 96% or more, 97% or more, 98% or more, 99% or more, 99.5% or more, or 99.9% or more to the specific nucleotide sequence or amino acid sequence or essentially comprises thereof and maintains the original functions and/or desired functions.

The polynucleotide of the present disclosure may have various modifications in coding regions within a range which does not change the amino acid sequence of the variant of the present disclosure, in consideration of degeneracy of codons or codons preferred in an organism in which the variant of the present disclosure is to be expressed. Specifically, the polynucleotide of the present disclosure may have, comprise, consist essentially of, or consist of a nucleotide sequence with homology or identity of 70% or more, 75% or more, 80% or more, 85% or more, 90% or more, 95% or more, 96% or more, 97% or more, 98% or more, and less than 100% to any one nucleotide sequence selected from the group consisting of nucleotide sequences of SEQ ID NOs: 20 to 38 (for example, the nucleotide sequence of SEQ ID NO:21, SEQ ID NO:27, or SEQ ID NO:33), but not limited thereto.

In addition, the polynucleotide of the present disclosure may comprise a probe that can be prepared from a known gene sequence, for example, any sequence capable of hybridizing with a complementary sequence to all or a part of the polynucleotide sequence of the present disclosure under stringent conditions without limitation. The "stringent condition" means a condition that allows specific hybridization between polynucleotides. For example, a condition of hybridizing polynucleotides with high homology or identity, polynucleotides with homology or identity of 70% or more, 75% or more, 80% or more, 85% or more, 90% or more, 95% or more, 96% or more, 97% or more, 98% or more, or 99% or more, and not hybridizing polynucleotides with homology or identity lower than that, or a condition of washing at a salt concentration and temperature corresponding to 60°C, 1×SSC, 0.1% SDS, specifically, 60°C, 0.1×SSC, 0.1% SDS, more specifically, 68°C, 0.1×SSC, 0.1% SDS, which is a washing condition of common southern hybridization, once, specifically, twice to 3 times may be listed.

Hybridization requires that two nucleotides have complementary sequences, but mismatches between bases may be allowed depending on the stringency of hybridization. The term, "complementary" may be used to describe relationship between nucleotide bases capable of hybridizing to each other. For example, in case of DNA, adenosine is complementary to thymine, and cytosine is complementary to guanine. Therefore, the polynucleotide of the present disclosure may also comprise not only a substantially similar nucleotide sequence, but also an isolated nucleic acid fragment complementary to the entire sequence.

Specifically, the polynucleotide with homology or identity to the polynucleotide of the present disclosure may be detected using a hybridization condition including a hybridization step at a Tm value of 55°C and using the afore-mentioned conditions. In addition, the value of Tm may be 60°C, 63°C or 65°C, but not limited thereto, and may be appropriately adjusted by those skilled in the art according to the purpose.

The suitable stringency to hybridize the polynucleotide depends on the length and degree of complementarity of the polynucleotide, and variables are well known in the art.

Other aspect of the present disclosure provides a vector comprising the polynucleotide of the present disclosure. The vector may be an expression vector to express the polynucleotide in a host cell, but not limited thereto.

The vector of the present disclosure may comprise a DNA product comprising a nucleotide sequence of a polynucleotide encoding the target polypeptide operably linked to an expression regulatory region (or expression regulatory sequence) suitable for expressing a target polypeptide in an appropriate host. The expression regulatory region may comprise a promoter that can initiate transcription, any operator sequence to regulate such transcription, a sequence encoding an appropriate mRNA ribosome binding site, and/or a nucleic acid sequence that regulates termination of transcription and translation. The vector may be transformed into a suitable host cell, and then be replicated or function independently of the host genome, and be integrated into the genome itself.

The vector used in the present disclosure is not particularly limited, and any vector known in the art may be used. Examples of the commonly used vector may include plasmids, cosmids, viruses, and bacteriophages in a natural state or a recombined state. For example, as the phage vector or cosmid vector, pWE15, M13, MBL3, MBL4, IXII, ASHII, APII, t10, t11, Charon4A, and Charon21A, and the like may be used, and as the plasmid vector, pDZ-based, pBR-based, pUC-based, pBluescriptll-based, pGEM-based, pTZ-based, pCL-based and pET-based and the like may be used. Specifically, pDZ, pDC, pDCM2, pACYC177, pACYC184, pCL, pECCG117, pUC19, pBR322, pMW118, pCC1BAC, or pDC24 vector, or the like may be used.

As one example, through a vector for inserting chromosome into a cell, a polynucleotide encoding a target polypeptide may be inserted into chromosome. Insertion of the polynucleotide into chromosome may be performed by any method known in the art, for example, homologous recombination, bout not limited thereto. A selection marker to confirm whether the chromosome is inserted may be further comprised. The selection marker is to select a cell transformed with a vector, that is, to confirm whether a target nucleic acid molecule is inserted, and markers which impart selectable phenotypes such as drug resistance, auxotrophy, resistance to a cytotoxic agent, or expression of a surface protein may be used. In an environment treated with a selective agent, only cells expressing the selection marker survive or show other phenotypes, so transformed cells may be selected.

In the present disclosure, the term, "transformation" means introducing a vector comprising a polynucleotide encoding a target polypeptide into a host cell or microorganism so that the polypeptide encoded by the polynucleotide is to be expressed in the host cell. As long as the transformed polynucleotide can be expressed in a host cell, all of them may be included regardless of whether they are inserted into chromosome of the host cell or located outside the chromosome. In addition, the polynucleotide comprises DNA and/or RNA encoding a target polypeptide. The polynucleotide may be introduced in any form, as long as it can be introduced into a host cell and expressed. For example, the polynucleotide may be introduced into a host cell in a form of expression cassette, which is a gene structure comprising all elements required for being expressed by itself. The expression cassette may commonly comprise a promoter operatively linked to the polynucleotide, a transcription termination signal, a ribosome binding site and a translation termination signal. The expression cassette may be in a form of an expression vector capable of self-replication. In addition, the polynucleotide may be introduced into a host cell in its own form and be operatively linked to a sequence required for expression in the host cell, but not limited thereto.

Furthermore, in the above, the term, "operatively linked" refers to that the polynucleotide sequence is functionally linked to a promoter sequence which initiates and mediates transcription of the polynucleotide encoding the target variant of the present disclosure.

Other aspect of the present disclosure provides a microorganism of the genus of *Corynebacterium,* comprising at least one selected from the group consisting of the protein variant of the present disclosure and the polynucleotide of the present disclosure.

The microorganism of the present disclosure may comprise the protein variant of the present disclosure, a polynucleotide encoding the protein variant, or a vector comprising the polynucleotide of the present disclosure.

In the present disclosure, the term, "microorganism (or strain)" includes all of wild-type microorganisms or microorganisms in which genetic modification occurs naturally or artificially, and may be a microorganism of which specific mechanism is weakened or strengthened due to reasons such as insertion of a foreign gene or enhancement or inactivation of activity of an intrinsic gene and the like, which is a microorganism comprising genetic modification for production of a target polypeptide, protein or product.

The microorganism of the present disclosure may be a microorganism comprising any one or more of the protein variant of the present disclosure, the polynucleotide of the present disclosure, and a vector comprising the polynucleotide of the present disclosure; a microorganism modified to express the variant of the present disclosure or the polynucleotide of the present disclosure; a microorganism (for example, recombinant microorganism) expressing the variant of the present disclosure, or the polynucleotide of the present disclosure; or a microorganism (for example, recombinant microorganism) having the activity of the variant of the present disclosure, but not limited thereto.

The microorganism of the present disclosure may be a microorganism having L-isoleucine productivity.

The microorganism of the present disclosure may have increased L-isoleucine productivity, compared to a microorganism of the genus of *Corynebacterium* which does not comprise any one selected from the group consisting of the afore-mentioned protein variant of the present disclosure and the polynucleotide encoding the protein variant (i.e., a microorganism which does not express a protein variant comprising an amino acid sequence in which the amino acid corresponding to the 27th position from the N-terminus in the amino acid sequence of SEQ ID NO:116 is substituted with an amino acid (for example, aspartic acid) different from the original amino acid, an amino acid sequence in which the amino acid corresponding to the 461st position from the N-terminus in the amino acid sequence of SEQ ID NO:117 is substituted with an amino acid (for example, glutamic acid) different from the original amino acid, or an amino acid sequence in which the amino acid corresponding to the 75th position from the N-terminus in the amino acid sequence of SEQ ID NO:118 is substituted with an amino acid (for example, valine) different from the original amino acid or a protein variant comprising any one amino acid sequence selected from the group consisting of SEQ ID NOs:1 to 19, for example, the amino acid sequence of SEQ ID NO:2, SEQ ID NO:8, or SEQ ID NO:14; or a microorganism that expresses the wild-type protein corresponding to the protein variant).

The microorganism of the present disclosure may be a microorganism naturally having L-isoleucine productivity, or a microorganism in which the variant of the present disclosure or a polynucleotide encoding thereof (or a vector comprising the polynucleotide) is introduced into a parent strain having no L- isoleucine productivity and/or L-isoleucine productivity is imparted, but not limited thereto.

As one example, the microorganism of the present disclosure is a cell or microorganism expressing the protein variant of the present disclosure, as transformed with a vector comprising the polynucleotide of the present disclosure or a polynucleotide encoding the variant of the present disclosure, and for the purpose of the present disclosure, the microorganism of the present disclosure may include all microorganisms capable of producing L-isoleucine by comprising the variant of the present disclosure. For example, the microorganism of the present disclosure may be a natural wild-type microorganism or a recombinant microorganism with increased L-isoleucine productivity by introducing the polynucleotide encoding the variant of the present disclosure into a microorganism producing L-isoleucine.

In one embodiment, the microorganism of the present disclosure may have increased L-isoleucine productivity compared to a microorganism of the genus of *Corynebacterium* that does not comprise at least one selected from the group consisting of the protein variants of the present disclosure and polynucleotides encoding the protein variants. The microorganism of the genus of *Corynebacterium* which does not comprise at least one selected from the group consisting of the protein variants of the present disclosure and polynucleotides encoding the protein variants may be a natural wild-type microorganism or a non-modified microorganism, and may also be referred to as a parent strain or host cell.

In the present disclosure, the term, "non-modified microorganism" does not exclude a microorganism comprising mutation that can occur naturally in a microorganism, and may mean a wild-type microorganism or natural microorganism itself, or a microorganism before traits are changed due to genetic mutation caused by natural or artificial factors. For example, the non-modified microorganism may mean a microorganism in which the protein variant described in the present description is not introduced or before introduced. The "non-modified microorganism" may be interchangeably used with "strain before modification", "microorganism before modification", "non-mutated strain", "non-modified strain", "non-mutated microorganism" or "reference microorganism".

In the present disclosure, the microorganism as the host cell (non-modified microorganism) may be: (1) a microorganism having a natural L-isoleucine productivity; (2) a microorganism having no or significantly low natural L-isoleucine productivity; and/or (3) a microorganism into which a mutation (transformation) has been introduced in the microorganism of (1) or (2) so as to have or enhance L-isoleucine productivity. In one specific embodiment, the microorganism as the host cell may be *Corynebacterium glutamicum* KCCM12739P (CA10-3101, US 2023-0098971 A1) or *Corynebacterium glutamicum* KCCM11248P (Korean Patent No. 10-1335789), but is not limited thereto.

In one specific embodiment, the microorganism (mutant microorganism or host cell microorganism) may be a microorganism in which the biosynthetic pathway of L-isoleucine is further enhanced in order to increase the production of L-isoleucine, but is not limited thereto.

In one embodiment, the microorganism may be a microorganism in which feedback inhibition of L-threonine dehydratase is released and/or feedback inhibition of homoserine dehydrogenase is released.

In one specific embodiment, the microorganism may be a microorganism in which feedback inhibition of L-threonine dehydratase is released.

In the present disclosure, the term "threonine dehydratase (EC 4.3.1.19)" refers to an enzyme that produces 2-ketobutyrate from threonine, is encoded by the *ilvA* gene, and is known to be subject to feedback inhibition by L-isoleucine. Microorganisms of the genus of *Corynebacterium* synthesize L-isoleucine through three intermediates using pyruvate and 2-ketobutyrate as precursors. The amino acid sequence of threonine dehydratase can be obtained from publicly available databases such as NCBI GenBank, or from US 2023-0098971 A1 and US 10982244 B2.

In one specific embodiment, the microorganism may be a microorganism in which feedback inhibition of homoserine dehydrogenase is released.

In the present disclosure, the term "homoserine dehydrogenase (EC 1.1.1.3)" refers to an enzyme encoded by the *hom* gene that catalyzes the synthesis of homoserine, and is known to be subject to feedback inhibition by isoleucine. The amino acid sequence of homoserine dehydrogenase can be obtained from publicly available databases such as NCBI GenBank or US 10982244 B2.

In one embodiment, the microorganism may be a microorganism in which feedback inhibition of aspartate kinase is released. In the present disclosure, the term "aspartate kinase (EC 2.7.2.4)" refers to an enzyme encoded by the *lysC* gene, and the amino acid sequence of aspartate kinase can be obtained from publicly available databases such as NCBI GenBank or US 10662450 B2.

In the present disclosure, the term "release of feedback inhibition" may refer to a state in which the activity of a polypeptide, protein, or enzyme is increased or enhanced compared with its intrinsic activity, or in which the activity is not inhibited compared with its intrinsic activity.

In one embodiment, the microorganism may further comprise one or more genetic mutations selected from the group consisting of: a mutation in the *hom* gene (R407H) (US 10982244 B2); mutations in the *ilvA* gene (T381A, F383A, and/or V323A) (US 2023-0098971 A1, US 10982244 B2); and a mutation in the *lysC* gene encoding aspartate kinase (L377K) (US 10662450 B2). However, the present disclosure is not limited thereto.

In one embodiment, the microorganism with increased L-isoleucine productivity (produced amount) of the present disclosure may have increased L-isoleucine productivity (produced amount) of about 1% or more, about 10% or more, about 20% or more, about 30% or more, about 40% or more, about 42.5% or more, about 42.75% or more, about 45% or more, or about 47.5% or more (the upper limit is not particularly limited, and for example, it may be about 200% or less, about 150% or less, about 100% or less, or about 50% or less), compared to a parent strain before mutation or to a non-modified microorganism, but is not limited thereto. In another embodiment, the microorganism with increased L-isoleucine productivity (produced amount) of the present disclosure may have increased L-isoleucine productivity (produced amount) of about 1.01 times or more, about 1.1 times or more, about 1.2 times or more, about 1.3 times or more, about 1.4 times or more, about 1.425 times or more, about 1.4275 times or more, about 1.45 times or more, or about 1.475 times or more (the upper limit is not particularly limited, and for example, it may be about 10 times or less, about 5 times or less, about 3 times or less, or about 2 times or less), compared to a parent strain before mutation or to a non-modified microorganism, but is not limited thereto.

The term "about" is a range including all of ±0.5, ±0.4, ±0.3, ±0.2, ±0.1, and the like, and includes all numerical values identical or similar to the numerical value appearing after the term of about, but is not limited thereto.

In one embodiment, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli*, *Corynebacterium imitans, Corynebacterium testudinoris,* or *Corynebacterium flavescens,* and more specifically, it may be *Corynebacterium glutamicum.*

In the present disclosure, the term, "weakening" of the polypeptide, is a concept including both reducing activity or having no activity compared to the intrinsic activity. The weakening may be interchangeably used with terms such as inactivation, deficiency, down-regulation, decrease, reduce, attenuation, and the like.

The weakening may also include cases where the activity of the polypeptide itself is reduced or removed compared to activity of the polypeptide possessed by the original microorganism due to mutation of a polynucleotide encoding the polypeptide and the like, cases where the overall polypeptide activity level and/or concentration (expression level) is lower than a natural strain in cells due to inhibition of expression or inhibition of translation into a polypeptide of a gene of a polynucleotide encoding the same, and the like, cases where expression of the polynucleotide is not done at all, and/or cases where there is no activity of the polypeptide even if the polynucleotide is expressed. The "intrinsic activity" refers to activity of a specific polypeptide originally possessed by a parent strain before trait changes or non-modified microorganism, when traits are changed due to genetic mutation caused by natural or artificial factors. This may be used interchangeably with "activity before modification". That activity of a polypeptide is "inactivated, deficient, decreased, down-regulated, reduced, or attenuated" compared to the intrinsic activity, refers to that it is lowered compared to the activity of a specific polypeptide originally possessed by a parent strain before trait changes or non-modified microorganism.

This weakening of the activity of the polypeptide may be performed by any method known in the art, but not limited thereto, and may be achieved by application of various methods well known in the art (for example, Nakashima N et al., Bacterial cellular engineering by genome editing and gene silencing. Int J Mol Sci. 2014;15(2):2773-2793, Sambrook et al. Molecular Cloning 2012, etc.).

Specifically, the weakening of the polypeptide may be
1) deficiency of the entire or a part of a gene encoding a polypeptide;
2) modification of an expression regulatory region (or expression regulatory sequence) so that expression of a gene encoding a polypeptide is reduced;
3) modification (for example, deletion/substitution/addition of at least one amino acid in the amino acid sequence) of an amino acid sequence composing the polypeptide so that the activity of the polypeptide is removed or weakened;
4) modification (for example, addition/substitution/addition of at least one nucleic acid base in the nucleic acid base sequence of the polypeptide gene to encode a polypeptide modified so that the activity of the polypeptide is removed or weakened) of a gene sequence encoding the polypeptide so that the activity of the polypeptide is removed or weakened;
5) modification of a base sequence encoding an initiation codon or 5'-UTR area of gene transcriptome encoding a polypeptide;
6) introduction of an antisense oligonucleotide (for example, antisense RNA) complementarily binding to transcriptome of a gene encoding a polypeptide;
7) addition of a sequence complementary to Shine-Dalgarno sequence in front of the Shine-Dalgarno sequence of a gene encoding a polypeptide so as to form a secondary structure incapable of attachment of ribosome;
8) addition of a promoter transcribed in the opposite direction at the 3' end of an ORF (open reading frame) of a gene sequence encoding a polypeptide (Reverse transcription engineering, RTE); or
9) adjustment of cellular localization of a protein (polypeptide); or
10) a combination of at least 2 selected from the 1) to 9), but not particularly limited thereto.

For example,
the 1) deficiency of the entire or a part of a gene encoding a polypeptide may be removal of the entire polynucleotide encoding an intrinsic target polypeptide in chromosome, replacement with a polynucleotide in which some nucleotides are deleted, or replacement with a marker gene.

In addition, the 2) modification of an expression regulatory region (or expression regulatory sequence) may be mutation occurrence on the expression regulatory region (or expression regulatory sequence) by deletion, insertion, non-conservative or conservative substitution or a combination thereof, or replacement with a sequence having weaker activity. The expression regulatory region includes a promoter, an operator sequence, a sequence encoding a ribosome binding site, and a sequence regulating termination of transcription and translation, but not limited thereto.

Furthermore, the 3) modification of a base sequence encoding an initiation codon or 5'-UTR area of gene transcriptome encoding a polypeptide may be for example, substituting it with a base sequence encoding another initiation codon with a lower polypeptide expression rate compared to the intrinsic initiation codon, but not limited thereto.

Moreover, the modification of an amino acid sequence or polynucleotide sequence of the 4) and 5) may be occurrence of mutation in the sequence by deletion, insertion, non-conservative or conservative substitution or a combination thereof, or replacement with an amino acid sequence or polynucleotide sequence improved to have weaker activity or an amino acid sequence or polynucleotide sequence improved to have no activity, but not limited thereto. For example, by forming a stop codon by introducing mutation in a polynucleotide sequence, expression of a gene may be inhibited or weakened, but not limited thereto.

The 6) introduction of an antisense oligonucleotide (for example, antisense RNA) complementarily binding to transcriptome of a gene encoding a polypeptide may refer to for example, the document [Weintraub, H. et al., Antisense-RNA as a molecular tool for genetic analysis, Reviews - Trends in Genetics, Vol. 1(1) 1986].

The 7) addition of a sequence complementary to Shine-Dalgarno sequence in front of the Shine-Dalgarno sequence of a gene encoding a polypeptide so as to form a secondary structure incapable of attachment of ribosome may make mRNA translation impossible or inhibit its speed.

The 8) addition of a promoter transcribed in the opposite direction at the 3' end of an ORF (open reading frame) of a gene sequence encoding a polypeptide (Reverse transcription engineering, RTE) may create an antisense nucleotide complementary to the transcriptome of the gene encoding the polypeptide to weaken the activity.

The 9) adjustment of cellular localization of a protein (polypeptide) may target a protein (polypeptide) to an intracellular specific organelle or specific intracellular space. For example, by addition or removal of a leader sequence functioning in targeting a protein (polypeptide), it may target it to periplasm or cytoplasm, but not limited thereto.

Such weakening of the activity of the polypeptide, may weaken the activity or concentration expression level of the corresponding polypeptide based on the activity or concentration of the expressed polypeptide in a wild-type or pre-transformed microbial strain, or increasing the amount of products produced from the corresponding polypeptide, but is not limited thereto.

In the present disclosure, the term, "enhancement" of the polypeptide activity, means that the activity of the polypeptide is increased compared to the intrinsic activity. The enhancement may be interchangeably used with terms such as activation, up-regulation, overexpression, increase and the like. Herein, the activation, up-regulation, overexpression, and increase may include showing activity which is not originally present, or showing activity improved compared to the intrinsic activity or activity before modification. The "intrinsic activity" refers to activity of a specific polypeptide originally possessed by a parent strain before trait changes or non-modified microorganism, when traits are changed due to genetic mutation caused by natural or artificial factors. This may be used interchangeably with "activity before modification". That activity of a polypeptide "is enhanced", "is up-regulated", "is overexpressed" or "increases" compared to the intrinsic activity, refers to that it is improved compared to the activity and/or concentration (expression level) of a specific polypeptide originally possessed by a parent strain before trait changes or non-modified microorganism.

The enhancement may be achieved by introducing a foreign polypeptide, or enhancement of activity of an intrinsic polypeptide and/or concentration (expression level). Whether the activity of the polypeptide is enhanced may be confirmed by the degree of activity, expression level of the corresponding polypeptide, or an increase of the amount of products released from the corresponding polypeptide.

For the enhancement of the activity of the polypeptide, various methods well known in the art may be applied, and there is no limitation, as long as the activity of a target polypeptide can be enhanced compared to a microorganism before modification. Specifically, genetic engineering and/or protein engineering well known to those skilled in the art, which are routine methods of molecular biology, may be used, but not limited thereto (for example, Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present disclosure may be
1) an increase of a copy number in cells of a polynucleotide encoding a polypeptide;
2) replacement of a gene expression regulatory region on chromosome encoding a polypeptide with a sequence having strong activity;
3) modification of a base sequence encoding an initiation codon or 5'-UTR area of gene transcriptome encoding a polypeptide;
4) modification of an amino acid sequence of the polypeptide so that the activity of the polypeptide is enhanced;
5) modification of a polynucleotide sequence encoding the polypeptide so that the activity of the polypeptide is enhanced (for example, modification of a polynucleotide of the polypeptide gene so as to encode a polypeptide modified to enhance the activity of the polypeptide);
6) introduction of a foreign polypeptide showing activity of a polypeptide or a foreign polynucleotide encoding the same;
7) codon optimization of a polynucleotide encoding a polypeptide;
8) modification or chemical modification by analyzing the tertiary structure of a polypeptide and selecting an exposed site;
9) adjustment of cellular localization of a protein (polypeptide); or
10) a combination of at least 2 selected from the 1) to 9), but not particularly limited thereto.

The 1) increase of a copy number in cells of a polynucleotide encoding a polypeptide may be achieved by introduction of a vector which can replicate and function regardless of a host, in which a polynucleotide encoding the corresponding polypeptide is operatively linked. Otherwise, it may be achieved by introduction of 1 copy or 2 copies or more of the polynucleotide encoding the corresponding polypeptide into chromosome in a host cell. The introduction into the chromosome may be performed by introducing a vector capable of inserting the polynucleotide into chromosome of the host cell into the host cell, but not limited thereto. The vector is as described above.

The 2) replacement of a gene expression regulatory region (or expression regulatory sequence) on chromosome encoding a polypeptide with a sequence having strong activity, may be for example, occurrence of mutation in the sequence by deletion, insertion, non-conservative or conservative substitution or a combination thereof so as to further intensify activity of the expression regulatory region, or replacement with a sequence having stronger activity. The expression regulatory region is not particularly limited thereto, but may comprise a promoter, an operator sequence, a sequence encoding a ribosome binding site, and a sequence regulating termination of transcription and translation and the like. As one example, the original promoter may be replaced with a strong promoter, but not limited thereto.

Examples of the known strong promoter include CJ1 to CJ7 promoters (U.S. Patent US 7662943 B2), lac promoter, trp promoter, trc promoter, tac promoter, lambda phage PR promoter, PL promoter, tet promoter, gapA promoter, SPL7 promoter, SPL13(sm3) promoter (U.S. Patent US 10584338 B2), O2 promoter (U.S. Patent US 10273491 B2), tkt promoter, yccA promoter, and the like, but not limited thereto.

The 3) modification of a base sequence encoding an initiation codon or 5'-UTR area of gene transcriptome encoding a polypeptide may be for example, substituting it with a base sequence encoding another initiation codon with a higher polypeptide expression rate compared to the intrinsic initiation codon, but not limited thereto.

The modification of an amino acid sequence or polynucleotide sequence of the 4) and 5) may be occurrence of mutation in the sequence by deletion, insertion, non-conservative or conservative substitution or a combination thereof, or replacement with an amino acid sequence or polynucleotide sequence improved to have stronger activity for the amino acid sequence of the polypeptide or a polynucleotide sequence encoding the polypeptide so as to strengthen the activity of the polypeptide, but not limited thereto. The replacement may be performed specifically by inserting a polynucleotide into chromosome by homologous recombination, but not limited thereto. The vector used then may further comprise a selection marker for confirming chromosome insertion. The selection marker is as described above.

The 6) introduction of a foreign polypeptide showing activity of a polypeptide may be introduction of a foreign polynucleotide encoding a polypeptide showing the same/similar activity to the polypeptide into a host cell. The foreign polynucleotide is not limited in its origin or sequence as long as it shows the same/similar activity to the polypeptide. The method used for the introduction may be performed by those skilled in the art by appropriately selecting a known transformation method, and by expressing the introduced polynucleotide in a host cell, a polypeptide may be produced and its activity may be increased.

The 7) codon optimization of a polynucleotide encoding a polypeptide may be codon optimization so that transcription or translation of an intrinsic polynucleotide is increased in a host cell, or codon optimization so that a foreign polynucleotide is optimized in a host cell to achieve transcription and translation.

The 8) modification or chemical modification by analyzing the tertiary structure of a polypeptide and selecting an exposed site may determine template protein candidates depending on the degree of similarity of sequences by comparing sequence information of a polypeptide to be analyzed, and confirm the structure based on this, and select an exposed site to be modified or chemically modified, and transform or modify it.

The 9) adjustment of cellular localization of a protein (polypeptide) may target a protein (polypeptide) into an intracellular specific organelle or specific intracellular space. For example, by addition or removal of a leader sequence functioning in targeting a protein (polypeptide), it may target it to periplasm or cytoplasm, but not limited thereto.

Such enhancement of the polypeptide activity means increasing activity or concentration expression level of the corresponding polypeptide based on the activity or concentration of the expressed polypeptide in a wild-type or pre-transformed microbial strain, or increasing the amount of products produced from the corresponding polypeptide, but is not limited thereto.

In the microorganism of the present disclosure, modification of a part or all of a polynucleotide (for example, modification to encode the afore-mentioned protein variant) may be induced by (a) homologous recombination using a vector for inserting chromosome into a microorganism or genome editing using genetic scissors (engineered nuclease, e.g., CRISPR-Cas9) and/or (b) treatment of light such as ultraviolet rays and radioactive rays and the like and/or chemical substances, but not limited thereto. The method of modifying a part of all of the gene may include a method for DNA recombination technology. For example, by injecting a nucleotide sequence or vector comprising a nucleotide sequence homologous to a target gene into the microorganism to cause homologous recombination, deletion of a part or all of the gene may be achieved. The injected nucleotide sequence or vector may include a dominant selection marker, but is not limited thereto.

In the microorganism of the present disclosure, the protein variant, polynucleotide and L-isoleucine and the like are as described in other aspects above.

Other aspect of the present disclosure provides a method of producing L-isoleucine, comprising culturing a microorganism of the genus of *Corynebacterium* comprising at least one selected from the group consisting of the protein variant of the present disclosure and the polynucleotide encoding the protein variant in a medium.

The method of producing L-isoleucine of the present disclosure may comprise culturing a microorganism of the genus of Corynebacterium comprising the protein variant of the present disclosure or the polynucleotide of the present disclosure or the vector of the present disclosure in a medium.

In the present disclosure, "culturing" means growing a microorganism of the genus of *Corynebacterium* of the present disclosure under an appropriately adjusted environmental condition. The culturing process of the present disclosure may be conducted according to an appropriate medium and culturing conditions known in the art. This culturing process may be easily adjusted and used by those skilled in the art depending on the selected strain. Specifically, the culturing may be batch, continuous, and/or fed-batch, but not limited thereto.

In the present disclosure, the term "medium" refers to a material in which nutrients required for culturing the microorganism of the genus of *Corynebacterium* are mixed as main components, and supplies nutrients and growth factors and the like including water which is essential for survival and growth. Specifically, the medium used for culturing the microorganism of the genus of *Corynebacterium* of the present disclosure and other culturing conditions may be used as long as it is a medium used for culturing a common microorganism without particular limitation, but the microorganism of the genus of *Corynebacterium* of the present disclosure may be cultured in a common medium containing a suitable carbon source, a nitrogen source, a phosphorus source, an inorganic compound, an amino acid and/or a vitamin and the like by adjusting temperature, pH and the like under an aerobic condition.

Specifically, the culture medium for the microorganism of the genus of *Corynebacterium* may be found in the document ["Manual of Methods for General Bacteriology" by the American Society for Bacteriology (Washington D.C., USA, 1981)].

In the present disclosure, as the carbon source, carbohydrates such as glucose, saccharose, lactose, fructose, sucrose, maltose, etc.; sugar alcohols such as mannitol, sorbitol, etc., organic acids such as pyruvate, lactate, citrate, etc.; amino acids such as glutamic acid, methionine, lysine, etc. and the like, may be included. In addition, natural organic nutrients such as starch hydrates, molasses, blackstrap molasses, rice bran, cassava, sugar cane residues, and corn steep liquor may be used, and specifically, carbohydrates such as glucose and sterilized pre-treated molasses (that is, molasses converted with reducing sugars) and the like may be used, and other carbon sources in an appropriate amount may be variously used without limitation. These carbon sources may be used alone or at least two kinds may be used in combination, but not limited thereto.

As the nitrogen source, inorganic nitrogen sources such as ammonia, ammonium sulfate, ammonium chloride, ammonium citrate, ammonium phosphate, ammonium carbonate, ammonium nitrate and the like; and organic nitrogen sources such as amino acids including glutamic acid, methionine, glutamine and the like, peptone, NZ-amine, meat extract, yeast extract, malt extract, corn steep liquor, casein hydrates, fish or degraded products thereof, defatted soybean cake or degraded products thereof, and the like may be used. These nitrogen sources may be used alone or at least two may be used in combination, but not limited thereto.

As the phosphorus source, potassium phosphate monobasic, potassium phosphate dibasic, or sodium-containing salts corresponding thereto, or the like may be included. As the inorganic compound, sodium chloride, calcium chloride, iron chloride, magnesium sulfate, iron sulfate, manganese sulfate, calcium carbonate and the like may be used, and in addition thereto, amino acids, vitamins, and/or appropriate precursors and the like may be included. These components or precursors may be added in a batch or continuous method. However, it is not limited thereto.

In addition, by adding a compound such as ammonium hydroxide, potassium hydroxide, ammonia, phosphoric acid, sulfuric acid and the like to a medium during culturing of the microorganism of the genus of Corynebacterium of the present disclosure by an appropriate method, the pH of the medium may be adjusted. Furthermore, during culturing, foam generation may be inhibited using an antifoaming agent such as fatty acid polyglycol ester. Moreover, in order to maintain the aerobic state of the medium, oxygen or oxygen-containing gas may be injected into the medium, or in order to maintain the anaerobic and microaerobic state, gas may be not injected or nitrogen, hydrogen or carbon dioxide gas may be injected, but not limited thereto.

The culture temperature in the culturing of the present disclosure may be 20 to 45°C, specifically, at 25°C to 40°C, 25°C to 40°C, 25°C to 37°C, 25°C to 35°C, 27°C to 40°C, 27°C to 37°C, 27°C to 35°C, 30°C to 40°C, 30°C to 37°C, or 30°C to 35°C, but not limited thereto. In the culturing of the present disclosure, the time of culturing may be about 10 to 160 hours, but not limited thereto.

The L-isoleucine produced by the culturing of the present disclosure may be released to a medium or remain in cells.

The method of producing L-isoleucine of the present disclosure, may further comprise preparing the microorganism of the genus of *Corynebacterium* of the present disclosure, preparing a medium for culturing the strain, or a combination thereof (in any order), for example, before the culturing.

The method of producing L-isoleucine of the present disclosure, may further comprise recovering L-isoleucine from the medium according to the culturing (medium in which culturing is performed) or the microorganism of the genus of *Corynebacterium.* The recovering may be further comprised after the culturing.

The recovering may collect targeted L-isoleucine using an appropriate method known in the art according to the culturing method of the microorganism of the present disclosure, for example, a batch, continuous or fed-batch culturing method, or the like. For example, centrifugation, filtration, treatment by a crystallized protein precipitator (salting out), extraction, ultrasonic crushing, ultrafiltration, dialysis, various kinds of chromatography such as molecular sieve chromatography (gel filtration), adsorption chromatography, ion exchange chromatography, affinity chromatography, and the like, HPLC or a combination of these methods may be used, and using an appropriate method known in the art, targeted L-amino acids may be recovered from a medium or microorganism.

In addition, the method of producing L-isoleucine of the present disclosure may additionally comprise a purification step. The purification may be performed, using an appropriate method known in the art. In one embodiment, when the method of producing L-isoleucine of the present disclosure comprises both the recovering step and purification step, the recovering step and purification step may be continuously or non-continuously performed regardless of the order, or may be performed by being integrated as one step, but not limited thereto.

In the method of the present disclosure, the variant, polynucleotide, vector and strain and the like are described in other aspects above.

Other aspect of the present disclosure provides a composition for producing L-isoleucine, the composition comprising: a microorganism of the genus of *Corynebacterium* comprising at least one selected from the group consisting of a protein variant of the present disclosure, a polynucleotide encoding the protein variant, and a vector comprising the polynucleotide; a medium in which the microorganism has been cultured; or a combination of two or more thereof.

The composition of the present disclosure may further comprise any appropriate excipient commonly used for compositions for producing amino acids, and such an excipient, may be for example, a preservative, wetting agent, dispersant, suspending agent, buffer, stabilizer, or tonicifying agent, or the like, but not limited thereto.

In the composition of the present disclosure, the variant, polynucleotide, vector, strain, medium and L-isoleucine and the like are as described in other aspects above.

According to other aspect of the present disclosure, the present disclosure may provide a method of increasing L-isoleucine productivity of the microorganism, a method of imparting L-isoleucine productivity to the microorganism, and/or a method of preparing a microorganism with increased L-isoleucine productivity, comprising introducing (for example, transforming) the afore-mentioned protein variant of the present disclosure, a polynucleotide encoding the variant, and/or a vector comprising the polynucleotide into a microorganism.

In the method of increasing L-isoleucine productivity of the microorganism, the method of imparting L-isoleucine productivity to the microorganism, and/or the method of preparing a microorganism with increased L-isoleucine productivity, the polynucleotide, recombinant vector, and microorganism and the like are as described above.

According to other aspect of the present disclosure, the present disclosure provides a use for producing L-isoleucine, and/or preparing a microorganism producing L-isoleucine, and/or imparting and/or increasing L-isoleucine productivity of a microorganism, of at least one selected from the group consisting of the afore-mentioned protein variant; the polynucleotide encoding the protein variant; the recombinant vector comprising the polynucleotide; and the microorganism comprising the protein variant, the polynucleotide encoding the protein variant, and/or the recombinant vector comprising the polynucleotide.

In the use of producing L-isoleucine, and/or preparing a microorganism producing L-isoleucine, and/or imparting and/or increasing L-isoleucine productivity of a microorganism, the protein variant, polynucleotide, recombinant vector, and microorganism, and the like are described above.

According to other aspect of the present disclosure, the present disclosure provides a composition, method, product, process, or use characterized by at least one element disclosed in the present disclosure.

### [ADVANTAGEOUS EFFECTS]

When culturing a microorganism of the genus of *Corynebacterium,* comprising the present variant of the present disclosure, high yield L-isoleucine production is possible compared to a microorganism having a conventional non-modified polypeptide.

### [MODE FOR INVENTION]

Hereinafter, the present disclosure will be described in more detail with reference to Examples. However, the following Examples are merely preferred embodiments for illustrating the present disclosure, and it is not intended that the scope of the present disclosure be limited thereto. Meanwhile, technical matters not specifically described in the present specification may be readily understood and practiced by those skilled in the art to which the present disclosure pertains or in related technical fields.

### Examples

### Example 1: Selection of mutant strain with increased isoleucine productivity by artificial mutation

### Example 1-1: Random mutagenesis through UV irradiation

To select a mutant strain with increased isoleucine productivity, an isoleucine producing strain, KCCM12739P (Korean Patent No. 10-2363913) was smeared on a nutrient medium comprising agar and cultured at 30°C for 16 hours. Colonies obtained in this way were irradiated with UV (Ultraviolet mutation) at a room temperature and random mutation was induced on the genome in the strain. The composition of the nutrient medium is as follows.

### <Nutrient medium (pH 7.2)>

Glucose 10g, meat extract 5g, polypeptone 10g, sodium chloride 2.5g, yeast extract 5g, agar 20g, urea 2g (on the basis of 1 liter of distilled water)

### Example 1-2: Selection of strain with improved L-isoleucine productivity

The secured colonies of about 3,000 were inoculated in a selection medium of 300 *µ*ℓ, respectively, and cultured in a 96-deep well plate at 32 °C, 1000 rpm for about 24 hours. In order to analyze the production amount of the produced L-amino acids in the cultured solution, the ninhydrin method was used (J. Biol. Chem. 1948. 176:367-388). After completing the culturing, 10 *µ*ℓ of the culture supernatant and *190 µ*ℓ of the ninhydrin reaction solution were reacted at 65 °C for 30 minutes, and then, the absorbance was measured with a spectrophotometer at a wavelength of 570 nm and about 30 colonies of the mutant strains showing an absorbance increased by 10% or more, compared to the absorbance of the control KCCM12739P strain, were selected. Other colonies showed similar or reduced absorbance compared to the control group. The composition of the selection medium is as follows.

### <Selection medium (pH 8.0)>

Glucose 10 g, ammonium sulfate 5.5 g, magnesium sulfate heptahydrate 1.2 g, potassium phosphate monobasic 0.8 g, potassium phosphate dibasic 16.4 g, biotin 100 *µ*g, thiamine-HCl 1000 *µ*g, calcium pantothenate 2000 *µ*g, nicotinamide 2000 *µ*g (on the basis of I liter of distilled water)

For the selected 30 strains, the ninhydrin reaction was repeatedly performed by the same method, and top 10 strains with improved L-isoleucine productivity compared to the KCCM12739P strain were selected.

### Example 1-3: Analysis of L-isoleucine productivity of selected mutant strains

In order to finally select strains with increased L-isoleucine productivity for the 10 strains selected in Example 1-2, a reproducibility test was conducted in a flask using the following mediums.

The above 10 strains and a control strain were inoculated into 250-mL corner-baffled flasks containing 25 mL of isoleucine production medium, followed by shaking culture at 32°C and 200 rpm for 60 hours. After completion of the culture, the concentration of L-isoleucine in the culture broth was analyzed by HPLC, and the L-isoleucine production levels of each mutant strain are shown in Table 2 below. The composition of the production medium was as follows:

### <Production medium (pH 7.2)>

Glucose 10%, yeast extract 0.2%, ammonium sulfate 1.6%, potassium phosphate monobasic 0.1%, magnesium sulfate heptahydrate 0.1%, ferrous sulfate heptahydrate 10 mg/L, manganese sulfate monohydrate 10 mg/L, and biotin 200 µg/L (per 1 L of distilled water).

**[Table 2]**

| Strain | L-isoleucine (g/L) |
|---|---|
| KCCM12739P | 2.0 |
| KCCM12739P_mt1 | 2.8 |
| KCCM12739P_mt2 | 2.6 |
| KCCM12739P_mt3 | 2.3 |
| KCCM12739P_mt4 | 3.4 |
| KCCM12739P_mt5 | 2.7 |
| KCCM12739P_mt6 | 3.1 |
| KCCM12739P_mt7 | 3.0 |
| KCCM12739P_mt8 | 3.0 |
| KCCM12739P_mt9 | 2.8 |
| KCCM12739P_mt10 | 2.4 |

Among the ten selected mutant strains, KCCM12739P_mt4 was finally selected as a strain exhibiting a significant improvement in L-isoleucine productivity.

### Example 2. Confirmation of mutation by whole-genome sequencing (WGS)

Whole-genome sequencing (WGS) was performed on the KCCM12739P_mt4 strain selected in Example 1, and by comparison with the parental strain KCCM12739P, genes in which nucleotide sequence variations caused changes in protein sequences were identified. The amino acid sequences of the protein variants and the nucleotide sequences of the corresponding genes are shown in Tables 3 and 4 below.

**[Table 3]**

| amino acid of wild-type strain | Protein mutation location | amino acid of variant strain | gene ID | Gene Name | Expressed protein | SEQ ID NO of the variant amino acid sequence | SEQ ID NO of the variant nucleotide sequence |
|---|---|---|---|---|---|---|---|
| E | 363 | D | NCgI0337 | WZZ | chromosome partitioning ATPase | 1 | 20 |
| E | 27 | D | NCgI0658 | IpdA | flavoprotein disulfide reductase | 2 | 21 |
| A | 182 | T | NCgI0674 | wbpC | acyltransferase | 3 | 22 |
| V | 23 | A | NCgI0686 | - | hypothetical protein | 4 | 23 |
| L | 1112 | F | NCgI0706 | - | Type II restriction enzyme, methylase subunits | 5 | 24 |
| I | 83 | V | NCgI0885 | cmt3 | corynomycolyl transferase | 6 | 25 |
| A | 114 | V | | | | | |
| E | 521 | Deletion* | NCgI1142 | narG | anaerobic dehydrogenase | 7 | 26 |
| D | 461 | E | NCgI1165 | atpD | ATP synthase F0F1 subunit beta | 8 | 27 |
| L | 305 | P | NCgI1253 | thiC | thiamine biosynthesis protein ThiC | 9 | 28 |
| E | 251 | G | NCgI1315 | uvrB | excinuclease ABC subunit B | 10 | 29 |
| S | 2 | G | NCgI1511 | ctaB | Protoheme IX farnesyltransferase | 11 | 30 |
| V | 81 | I | NCgI1588 | - | hypothetical protein | 12 | 31 |
| T | 56 | A | NCgI1815 | - | hypothetical protein | 13 | 32 |
| V | 41 | A | | | | | |
| T | 36 | A | | | | | |
| L | 20 | V | | | | | |
| K | 16 | E | | | | | |
| E | 15 | D | | | | | |
| G | 75 | V | NCgI1859 | fruR | transcriptional regulator of sugar metabolism | 14 | 33 |
| S | 164 | P | NCgI1874 | miaB | (dimethylallyl)aden osine tRNA methylthiotransferasse | 15 | 34 |
| A | 268 | S | NCgI2245 | - | Na+-dependent transporter | 16 | 35 |
| N | 284 | D | NCgI2580 | mutY | A/G-SPECIFIC ADENINE GLYCOSYLASE | 17 | 36 |
| G | 862 | V | NCgI2629 | - | hypothetical protein | 18 | 37 |
| R | 14 | W | NCgI2863 | cgtR9 | Two-component system, response regulator | 19 | 38 |

| | | | | | | | |
|---|---|---|---|---|---|---|---|
| * Deletion: A mutation in which substitution in the nucleotide sequence of a gene changes a codon originally encoding an amino acid into a stop codon, resulting in truncation of the amino acid sequence from that position. | | | | | | | |

**[Table 4]**

| SEQ ID NO | Sequence (amino acid sequence: N-terminus→ C-terminus; nucleotide sequence: 5'→3') |
|---|---|
| 1 | MELREYATILMKNWVLIVIASILGIAAGAGFSLLATPEYQSRTQLYVSVRSGAGT TSDMVQGANFSRQIVNSYVDVIKTGVVLEPVVDELGLELTANQLSSHISAASPA DTALINITASSPSPQQAAEIANAVGESFKNVIQTELEPDSGDGMSPINLTTTQVA LEPSSPVSPNVMMNILLGLLVGLAIGVGIAVLRAALDTRIHSLRDIEEVTDKPLLG GIIADSEVEKHPLIIKHKPHSPIAESFRALRTNLQFLNVGGSSSVFVISSANPGE GKSTTSVNLALALAEAGSRVALIEADLRLPRVSKYLGVEGNAGLTDILIGKAEVN DVLQRWGRTQLYYLPAGRIPPNPSELLGSAEMDKVIAELEESFDYVIIDAPPAL AVTDAAVIGHGKAGILIAVSAGSTKKPELEATLSTLENADANVVGVVATMLPPK SVAGYGYGNYGYGDTSKINAPKPDNTELTTTDASKANNEQ |
| 2 | MAKRIVIIGGGPAGYEAALAGAKYGADVTVIEDVGVGGSAVTMDCVPSKSFIA GTGIKTDLRRADDMGLNRGLGKAHLEIDALNIRVKDLAKAQSEDILGQLQRSDV RMINGVGRFDDYNTKQTTHYIKVTHSDGSEETVECDLVLVATGATPRILKGAE PDGERILTWRQVYDIEELPTHLIVVGSGVTGAEFVSAFAELGVKVTMVASRDRI LPHDDADAADVLETVLAERGVSLEKHARVESVTRTEDGGVCVRTADGREIYG SHALMTVGSIPNTADLGLENIGVELAPSGHIKVDRVSRTNIPGVYAAGDCTDLF PLASVAAMQGRIAMYHALGEGVSPIRLKTVATAVFTRPEIAAVGITHAQVDSGE VSARVIVLPLATNPRAKMRSLRHGFVKLFCRRNSGLIIGGVVVAPTASELILPIA VAVTNRLTVADLADTFAVYPSLSGSITEAARQLVQHDDLG |
| 3 | MGTARGLSQVLKEARQGGKPTSATQKVPRSGYRYDLDGLRGIAIAFVVLFHVF VGKVSGGVDVFLLLSGYFFLGSQLRYADRPDSSINPWWPIWRTLRRLLPALVL VLGVSMVLILAWVPRLQRIEIANQAVASLFYVQNWELASQGAAYGAASAEVSP FQHLWSMAVQGQFYLFAILLSMTIILIRRYRPEYSAVRLATPVLAVLTSVSFFSAI LWHFIDQSVNYYSTFTRFWELGLGALLVLHAPRILISAKTKSILAAVGLFMVLST GFFMDGAETFPGFPALYPILGACLVILGDGKISVFLSRKWMLWLGDIAYPLYLW HWPLLIIFTALFNQEEPSIWLGIAVIMLSLGLAQLTNKYVEKPMQQQGKRPLFR ESRSADALQKLRSSRPARMRAYAGIAVVAVGFALTSTPQVLYQRVLDTQMSD LDPEIYPGALSLAGFDAPEVKPKPNPYVLADTVSPAWAKGCMSVFGDDPSEL APDNYDEDYCAFGNPNSPVEVYLVGGSHAEQWMAPLDALGKQHNFKVIPLV RQSCPTFVVELDGIFSDDCIEFNEKVIERLAEVKPDLVVSNSTRPLLEKGRGIDE VPESYITLWDFLEAEDIPFMGLRDNPWFIQPGGEGWMVSQCYEEEQSLVDCS ITKDNFYAPVDPAEKELAIRNNMLAVDTSRWFCPDDLCIPVIGNVYVYRDGNH MSDEYAQSLAPFLWSNMRRLLVA |
| 4 | MSTAYFIAHGNDTYRPTEHSSGAWRDDELHLAPVAGLVIHHMERWRREVVG DALVFSRFSLEVLGQIARDDVTLRTEIVRPGRTIELIETVAEINGRVTIRARAWLL KTSDLAHISGDTFEALPSMTELADVKSAFQWSGGFMDSIVFVEDAEKIPGKNR AWITGDVQLVDGEDSEPLAEFSKFLDVANGVAVREDPKTWMFPNVDLTFHLF RQPVGTKVGFDTRAAFGPNGIGLTTSVIHDAEGPVGTLNQSLTVRPIAELNK |
| 5 | MSAFDSILNVEEWISDHYLTNDDAKGASFSKRVRERIKEWKTTEDATQQSGPL TRFSSNRLQLQHALSELDDATTAASLVASALGYGVPSARHAQRGSDTISYSS WVGNAGSVEFLAATPAESFEENFRSLPLEPVAVNDKPQDITAAKLVGQIFLSD TPPAFVVITAGKWVVLAERETWPLGRHLAIDISLVVERNDTKAQGEMQQTVVA LARENTERAADGTTWWEETIEQSREHAVKVSGELRSAVRESIEILGNDVLTRY EAKELSTAEIDGGELAKQSLRYLYRILFLLFAEASPELEILPTGTPEYDEGYGLA RLRELILNPPVTQHARTGTHLYDSLQLLFTLVDKGHHPTDAKAVAFDAEAGEE GLHFRNLSADLFLPAATELIDRVGLSNEALNKVLENLLLSRVQSGKDRGFISYA TLGVTELGQVYEGLMSYTGFIAQEDLFEVAPHGKADKGSWMLPVSKADEVPA DSFIEVDQEAPGGGVIKVRKRHPRGSFVFRQSSRDRERSASFYTPQVLTSFTV TQAIEELQASKRITTANDVLSLTICEPAMGSGAFAVEAVRQLAELYLELRQEEL EQQIPAEDRAKELQKVKAHIALHQVYGVDLNSTAVELAEISLWLDTMNAEMDA PWYGLHLRNGNSLVGATRSLYAPSLLNKKAWLTATPTRYRLDDIAQAIDENKA EPLFNHGIHHFLLPSTGWGATADAKDLKDLMATEIKELKSWRTSIRASLSKTQI KQLNNLALRVETLWRFVLMRIRIAESQISRSTTLWGQEPAEVSEVVTREQIEQD LFGNIDGAYNRLRLVMDAWCALWFWPLDAVATAEHPERPALPDLDEWLATLT EILGIDLPLKSKNENQIVLGPDTNWLAINDAEATDLGFSGALSFERVSANHPWI NVARQVAKQQSFFHWDLDFAHVFAKGGFDLQVGNPPWVRPDVNFEDLLAEH DPWWAVMSKPTQASKKERQKNFHNNPKSLEHVVSGAGETVATSAILGSVTLY PHLKDQRPDLYRGFMEKTWSNASPAGAISLIHPESHFTEKKAAPLRRGAYERL RRHWQFINELILFDVHDLVKYGVHVYGAPQESINFLSAASLYHPQTVLDSFDH DGSGNLPGLKDDNGNWDRRPHKDRIQLVNADTLTVWKSILEDEQTPYLDTRM VYTVNTEAAAVLEKLASAPRIKELGLQFSSGWNETTDKKKGYFDVGWGYPAS WSDAILQGPHLGVATPMIKQPNPTMKHNQDWSEIDFEAIPANFIPATAYQPDR QTKPTYDADYGTWTFGDKQVPVADTFRIAWREMAATTGFRTVYPSVIPPGAN HVHTVNSAASRSNLKTILVGAQLGAILSDYFARSSGSSHIFNDIVRKIPLPNFTS LEKQFARTYLRLNCLTSAYAPLWEEITGEPWDVQVPLRNAEQRRAAQNDIDA MVALSLGISADELCMIYRTQFPVMRRYDQEDHFDANGRKVPKEIIKLQQKLKD GQELSVEKRTWVHPQSEVSYTFEYPFRVLDREADLRAAYAKFENQLKEP |
| 6 | MRKGISRVLSVAVASSIGFGSVLSGTGIAAAQDSAFDYGMDPSMNYNPIDDIK DRPQGLSNLPYFGSKLTSWGSSDATASSGVVTSALPQYTDPRYPLGKDDLPK ATIDMEPEVLARLERFVGVDGDRIRQINAYSPSMGRTIPLVWVVPEDNTVPGP TVYALGGGDGGQGGQNWVTRTDLDELTSENNINLIMPMLGSFSFYADWAGE SESMGGAQQWETFLMHELPEPLEAAIGADGQRSIVGMSMSGGSVLNFATHD PNFYSSVGSFSGCAETNSWMGRRGIAATAYNGNVVPEQIFGEVDSDYSRYN DPLLNAAKLEEQDNLYIFAGSGVFSELDVIGDNAPIDEDAFKNRVLVGFEIEAM SNTCTHNLKAATDQMGIDNINYDFRPTGTHAWDYWNEALHRFFPLMMQGFG LDGGPIPIYNPNGVTSSESSSELSSDVSLGTVIGSVAGSSGSSAGSSVREFIAG SSGSSQSAGSFYE |
| 7 | MTTTTSSGKSSEKINPLFKLGSFLRKGTVGSEGQQIFLQGGRQADVFYRNRW AFDKVVRSTHGVNCTGSCSWKVYVKDGVITWESQAVDYPTTGADMPDYEPR GCPRGASFSWYTYSPTRIRYPYIRGVLVDMYREAKERLGDPVLAWRDIVETPE KRKAYVSQRGKGGLIRVQYEEAMEIAAAAHVYTIRQYGPDRIHGFTVIPAMSQ VSYGAGTRFLQMIGGVALSFYDWYADLPPASPQTFGDQTDVPESGDWYNSS YLMMWGSNIPVTRTPDSHFMVEARYKGTKVVVVSPDFADSTKFADEWARIHP GTDGALAFAMGHVILKEFHVDKKTPYFMDYMRKYTDSPFLVELDEHGDGTYT PGKFLTADRAADISPALAATPNATHRLLVLQKDGSVVDPGGTVADRWGEEGM GKWNLRLDGVDPVMTIADVQTDTETAEVLFPRFDLPATATQEGPIGAGTISRG VPTITLNGRKYTTVFDVLLAHYGVNREELNLPGEWPKDFQDPVMGTPAWQE |
| 8 | MTTALEEQNAQQAATAGRVVRVIGAVVDVEFPRGELPALYNALTVEVTLESVK KTVVLEVAQHLGDNLIRTIAMAPTDGLVRGAAVTDTARPISVPVGDVVKGHVF NALGDCLDDVSLNNNPEIERWGIHREPPSFDQLEGKTEILETGIKVIDLLTPYVK GGKIGLFGGAGVGKTVLIQEMITRIAREFSGTSVFAGVGERTREGTDLFLEMEE MGVLQDTALVFGQMDEPPGVRMRVALSGLTMAEYFRDVQNQDVLLFIDNIFR FTQAGSEVSTLLGRMPSAVGYQPTLADEMGVLQERITSTKGRSITSLQAVYVP ADDYTDPAPATTFAHLDATTELDRSIASKGIYPAVNPLTSTSRILEPAIVGERHY EVSQRVIGILQKNKELQDIIAILGMDELSEEDKITVARARRIERFLGQNFFVAEKF TGLPGSYVPLTDTVDAFERICNGDFDHYPEQAFNGLGGLDDVEAAYKKLTGK |
| 9 | MTPTQNEIHPKHSYSPIRKDGLEVPETEIRLDDSPSGPNEPFRIYRTRGPETNP KQGLPRLRESWITARGDVATYQGRERLLIDDGRSAMRRGQASAEWKGQKPA PLKALPGKRVTQMAYARAGVITREMEFVALREHVDAEFVRSEVARGRAIIPNN VNHPESEPMIIGRKFLTKINANIGNSAVTSSIEEEVSKLQWATRWGADTVMDLS TGDDIHTTREWIIRNSPVPIGTVPIYQALEKVNGVAADLNWEVFRDTIIEQCEQG VDYMTIHAGVLLAYIPLTTRRVTGIVSRGGSIMAGWCPAHHRESFLYEHFDELC EIFAQYDVAFSLGDGLRPGSLADANDAAQFAELKTIGELTQRAWEYDVQVMV EGPGHVPLNMIQENNELEQKWAADAPFYTLGPLVTDIAPGYDHITSAIGAAHIA MGGTAMLCYVTPKEHLGLPNRDDVKTGVITYKLAAHAADVAKGHPGARAWD DAMSKARFEFRWNDQFALSLDPDTAIAYHDETLPAEPAKTAHFCSMCGPKFC SMRISQDIRDMFGDQIAELGMPGVGDSSSAVASSGAREGMAEKSREFIAGGA EVYRR |
| 10 | MLARCFAAGRNVSTLWRILECMAFAAEHPVLSHSEHRPVGEIERSDDKFVVVS EFEPAGDQPAAIKELDERLDRGERDVVLMGATGTGKSATAAWLIEKQQRPAL VMAPNKTLAAQLANELRQLLPNNAVEYFVSYYDYYQPEAYIAQTDTYIEKDSSI NEDVERLRHSATSSLLSRRDVVVVSSVSCIYGLGTPQSYLDRSVVLNVGEEID RDRFLRLLVDIQYERNDVGFTRGAFRVKGDTVDIIPAYGELAVRIEFFGDEIDAL YYIHPLTGDTIRQVNEIRIFPATHYVAGPERMEKAVADIKAELEVRLADLENRGK LLEAQRLRMRTEYDLEMIEQVGFCSGIENYSRHIDGRGEGTAPATLIDYFPEDF LTIIDESHVTVPQIGGMFEGDMSRKRNLVEFGFRLPSAMDNRPLTWEEFDERR GQTVFMSATPGKFEIAAADGEFVEQVIRPTGLVDPKVTVKPTKGQIDDLIHEIR QRTDKDERVLVTTLTKKMAEDLTDYLLENGIRVRYLHSDIDTLQRVELLRQLRL GEYDVLVGINLLREGLDLPEVSLVAILDADKEGFLRSTTSLIQTIGRAARNVSGE VIMYADKITDSMQYAIEETDRRREKQVAYNKEHGIDPQPLRKKIADILDQVYDN SADGAGPSASGDAAVVAKPDVSSMPAKEVQKLIDDLSAQMAAAARELKFELA GRLRDEIFELKKELRGIKDAGI |
| 11 | MGDLKMQRSGGEPLDTIKAYIALTKPRVIELLLVATIPTMLQAERGENNIVLILLT VFGGWMGAAAANTFNMVADSDIDQRMGRTRARPLVRHTVSNRDASIFAWVL TVASFLWLWLLCDSMLAGIFVLITIFFYIFVYTKWLKRRTHMNIVWGGAAGCMP VLVGWAVIVDQFEPGVPQQWWQAIVLFMVIFFWTPPHTWALAMKYREDYKAA GVPMLPVVRTPVQVTAQIVWYSVATVLTTFLLIPATGWIYAAIAVISGVTFLFMAI KLHLGIKNGGKVKPLKLFILSNNYLAVLFVALSVDAVLGLETIGEMLGWTTTFF |
| 12 | MKLFSRTSLVALGTAAAITLSGVTAPAFADEDSNAAVSALKTAEDNTPEAPGAS TPLKLEQPGTITGVPGKAITPVTVKVIAGEAESFTSDNLPSGLLIDNTGKITGTPK KEFTGSAKIIAKNEAGVEAEVYVNFDFNEEPSSEEPSSGSSDTDNIENWIKIITA VIGALTTILTFSTKLDSFLK |
| 13 | MRSDVIELPEGVSKDEADQVEVAEARLNEGARLMAATGCEAMWPTGFSVCG RILDAYRQVGGQLSWLGPPKSNELTNPDGVGKRSEFFGGAIYWHPDTGAYAV TLDGLRQWGTLNWESGPLGYPTSGPMDTNYPLTQRQTFQGGDNYYNPLTG GAVWGDIKQRYEELGGSNHAIGIPITNELPSGTEYFYNNFSNGTISWRNDRQT RFMYLATQRVWDALGRETGRLGFPEADETPEVSGLFHVVNFAERGVIAWNGI LGARELYGDVYSLWLQYQNTDTPLGWPIPSLTSLNESLEQEFTRGVVLGSGD ALTWIPDDEERSLEDFLPIGSSGSSSSSQEMTLFSQRAQYVDCKNLPDLDEQR KTENNIEKNGGPIKKEYSSRGFPTEFRFVVRKGHYDRYRNEGWGYLKNYCKH NFANHAMAEAVVDKAVIDYGSSPGTSYYKFEKTVYFLDCRTYTFNKNSGCKE MHAPQWVTIIYNPHTFTGANSNRPKGVISAWCNSTPPGGIEHEPEISQCPDHV NLYNKLRI |
| 14 | MVSQTERQHAIASLLAPTGAVSVGDLAEHFHVTTETVRRDLRIMESLGLLQRV HGGAISPEPMGTSPPRLKPALVKGMPPEPRVLELAETAVSLITPLARSIFLDSG LACTAIATVLGDPPEDARWTVVTSSPGAVIALSATDATSTVVLHGQVHGNCSSI IGSTAVDMISQLRADIAFVEVDAIQSDTSLCTFFPETIPIKQAMIKNAAFTVAVLS PRSPQDQELQLLKHPFSTLADFDALVTDDHTLDFPVLPDHNFQVVTP |
| 15 | MTQQLNHAKVNQHPGQATLPETAEGQVRTYEVKTYGCQMNVHDSERLSGLL EEAGYVAAPEDTTPDLVVFNTCAVRENADMRLYGTLGNLRSVKEKNPGMQIA VGGCLAQKDKDTVVKKAPWVDVVFGTHNIGSLPTLLQRAEHNAQAEVEIVDSL EQFPSVLPAKRESAYAGWVSVSVGCNNTCTFCIVPSLRGKEQDRRPGDILAE VQALVDQGVTEVTLLGQNVNAYGVNFVDPELERDRSAFSKLLRACGEIEGLER VRFTSPHPAEFTSDVIDAMAETPNICPQLHMPLQSGSDKVLKEMRRSYRSKKF LSILDEVRAKIPHASITTDIIVGFPGETEEDFQATLDVVKKARFTSAYTFQYSPRP GTPAAEYENQLPKEVVQERYERLMVVQEQVCEEENQKLIGTTVELLVQAGGG RKNDATKRMSGRARDGRLVHFAPEGDIDGEIRPGDFVTVTVTEAKPFFLIADS GVQTHRRTKAGDNSAVGQVPTTAPIGVGLGLPQIGAPKVAPATESACCSIN |
| 16 | MSKTEEGRSAAIIIYAFPTFILLGAIIAFIFPEPFIPLTNYINIFLTIIMFTMGLTLTVP DFQMVLKRPLPILIGVVAQFVIMPFLAIVVAKMFNLNPALAVGLLMLGSVPGGT SSNVIAFLARGDVALSVTMTSVSTIVSPIMTPFLMLMLAGTETAVDGGGMAWT LVQTVLLPVIIGLVLRVFLNKWIDKILPILPYLSILGIGGVVFGAVAANAERLVSVG LIVFVAVIVHNVLGYVVGYLTGRVFKFPEAANRTMAIEIGTQSSGLASGMAGRF FTPEAALPGAVAALVHNITGAVYVGLVRNRPLTKASRKKESVAVSS |
| 17 | MSFTAFQTALLVWFRANARDLAWRDPNTSAWGILLSEVMSQQTPVARVEPIW REWMEKWPTPEDFANASTDEILRSWGKLGYPRRALRLKECAEVIVEKHAGEV PDTVEALLALPGIGDYTARAVAAFHFGQRVPVVDTNVRRVYQRAVAGRYLAG PAKKQELIDVSLLLPNTHAPEFSAAIMELGALICTATSPKCDTCPLLDQCQWQK LGCPSPSEEELASAKKRVQKFVGTDRQVRGLIMDVLRNATAPVPLSAIDVVWP DDAQRSRALFSLIEDGLAEQDEAGYFHLPR |
| 18 | MVNTLNSKTVNVPRFARGVVAAATALFFGALVSLAPSALAQEPPAVEAGASGS LSNLGACIADKGTLDVIIMIDETESLIHEARDGVVNANEPGADAQHHRVPAAQS FVDELLAKQSDGDLNTRIRVAGFGQTYKSGATDPDNYGAWTQLDASTVGGV QDEISRFADRTQEQYTNYASAIEGAYQDFTRSGSEDACRMLVTFTDGALTAQ EGADVAEAALCAPGGVTDRLRSAGITHIGIGLSAPTNPSDFSLLRGTTAGGGT CGVEPANGAFFPADNVGGLFAAFREALAIGGETIGETRAGDPFSFTLDNSVNS VRFTAIAKDDLGPNAHLVLTAPNGETVELKDSGSSVANSTDVSWEAESSPVK MADGSLNLQQGGDWKGVWQIQFQGIDPAAVDGRVFNSVEIQPDLQLVFSGG DSTSGALNLRDDQQLNMQLVGRDGQPRILEGSALVDLGFTRADTGEFAPLAQ GIDISGGELSFPLDTISQLPAIGTVEARTTITTAGVDDLPGTTLSPILNTTRITITQ RDMPQLPASVRFTADEDVVTVDIPITGPGKVWIAPGTQLSGVLPDGVDGIAAS STFDSPDNALVLGLDEQGTIPVELTVSDLRDGLVNGSIPLQISNAEGANETSVD LPTEGTLSVPINASTFALAFILALVLSLLIPLLILYIVRFLSAKVPSSAMSGVRIPVE FSGEALRYAGSTMPDLASQTTATKQVVVHGDTFNVEGHKLKVQRFQLNPIAS PAVIVQTDPSISFDGKQKGTQAKLPLAVQGSWFLTASGADPSKMELIALTNLPL EQGQIDRMIAGITSKAPDRARELQKLLDDAATSQPAKVPPRAPAAQGHVEKQA PSFGTGSGGVFGSSNGGGFGSGSGSNDTNGGFGSSGGFGAR |
| 19 | MADRTPTTATPPGWVLVVDDEQPLAQMVASYLIRAGFDTRQAHTGTQAVDEA RRFSPDVVVLDLGLPELDGLEVCRRIRTFSDCYILMLTARGSEDDKISGLTLGA DDYITKPFSIRELVTRVHAVLRRPRTSTTPPQVTTPLIVGDLILDPVAHQVWVGE TTVELTRTEFELLVALALRPGQVLTRHDLITEVWDTTWVGDERIVDVHIGNLRR KLGTDTRGRGFIDTVRGVGYRVGQP |
| 20 | ATGGAATTAAGGGAATACGCGACAATCCTGATGAAGAATTGGGTGTTGATC GTTATCGCATCCATTCTTGGAATTGCAGCGGGCGCTGGTTTTTCCCTTCTA GCTACACCGGAGTATCAGTCACGTACTCAGTTGTATGTATCGGTGCGGTC GGGGGCTGGGACAACCTCTGACATGGTTCAGGGTGCTAACTTTTCGCGTC AGATTGTGAATAGTTATGTTGATGTCATTAAGACGGGTGTTGTTCTAGAGC CGGTTGTTGATGAGCTTGGTCTGGAGTTGACGGCTAACCAGTTGAGTTCTC ATATCAGTGCGGCTTCTCCTGCTGATACTGCGTTGATCAACATCACTGCTT CTAGTCCTTCTCCTCAGCAGGCGGCTGAGATCGCCAATGCAGTGGGGGAG AGTTTCAAGAATGTGATTCAGACTGAATTGGAACCAGACTCCGGCGATGGC ATGAGCCCGATTAACCTGACTACTACTCAGGTGGCTCTGGAACCATCTTCT CCGGTCAGTCCTAATGTGATGATGAATATCCTCCTCGGCCTACTTGTAGGT CTTGCAATAGGTGTTGGTATTGCAGTGCTTCGCGCGGCTTTGGATACTCGT ATTCATTCCTTGCGCGATATTGAAGAGGTTACTGATAAGCCACTTTTGGGC GGAATCATTGCGGATTCTGAAGTTGAGAAGCACCCGTTGATCATTAAGCAC AAGCCGCATAGTCCTATTGCGGAGTCCTTTCGTGCGTTACGTACTAACCTG CAGTTCCTTAACGTCGGCGGCTCATCTTCAGTATTTGTTATCTCCTCTGCTA ATCCTGGTGAGGGTAAGTCAACCACTTCTGTAAACCTAGCTTTGGCGCTTG CAGAGGCCGGCTCCCGTGTAGCGCTGATCGAAGCTGATCTTCGCTTGCCA CGAGTGAGCAAGTACCTCGGAGTTGAGGGCAACGCAGGTCTGACTGACAT TCTCATTGGCAAGGCCGAGGTTAATGATGTGTTGCAGCGTTGGGGTAGGA CTCAGTTGTACTATCTCCCGGCAGGGCGCATTCCGCCGAACCCGAGTGAG TTGCTTGGTTCAGCTGAGATGGACAAGGTCATTGCGGAGCTTGAGGAAAG CTTTGATTATGTGATCATCGATGCCCCTCCGGCGTTGGCGGTTACCGATGC TGCAGTTATTGGTCATGGCAAGGCTGGCATCCTGATTGCGGTCTCCGCAG GTTCTACAAAGAAGCCTGAGTTGGAAGCTACGCTGTCCACGCTTGAGAAT GCGGATGCCAATGTTGTTGGCGTTGTCGCTACGATGCTCCCGCCTAAGTC TGTGGCTGGTTATGGCTACGGAAATTACGGCTACGGCGACACCTCCAAAA TCAATGCCCCTAAGCCCGACAACACCGAACTAACCACCACCGATGCTTCC AAGGCCAACAATGAGCAATAG |
| 21 | ATGGCAAAGAGGATCGTAATTATCGGCGGTGGACCTGCAGGCTATGAAGC CGCACTCGCAGGCGCTAAATACGGTGCAGACGTTACCGTTATTGAAGATG TCGGAGTTGGCGGATCCGCAGTCACCATGGACTGTGTACCTTCAAAGTCC TTCATCGCTGGTACCGGTATCAAAACCGACCTCCGACGTGCTGATGACAT GGGACTTAACCGTGGGCTTGGAAAAGCACACCTAGAAATCGATGCACTGA ACATCCGTGTGAAGGACCTTGCGAAAGCACAGTCCGAAGATATCTTGGGC CAGCTGCAGCGCTCAGATGTCCGCATGATTAACGGTGTGGGCCGCTTTGA TGATTACAACACCAAGCAAACCACCCACTACATTAAAGTCACCCACAGCGA TGGCTCCGAAGAAACCGTTGAGTGCGATCTGGTGCTGGTTGCAACTGGTG CAACCCCCCGCATTCTTAAAGGTGCAGAGCCAGACGGCGAGCGCATCTTG ACCTGGCGTCAGGTCTACGACATTGAAGAACTCCCCACCCACCTTATCGT GGTTGGTTCCGGTGTGACCGGTGCGGAATTTGTCTCTGCGTTTGCTGAAC TCGGCGTCAAAGTCACCATGGTGGCATCCCGTGACCGCATTTTGCCTCAC GATGACGCAGATGCCGCAGACGTGCTGGAAACCGTTCTGGCTGAGCGCG GAGTATCCCTGGAAAAGCATGCCCGCGTGGAGTCTGTCACCCGCACCGAA GACGGTGGCGTGTGTGTTCGCACTGCTGACGGACGAGAAATCTACGGTTC TCACGCGTTGATGACTGTTGGTTCCATTCCAAACACGGCAGATCTTGGCCT GGAGAACATCGGTGTTGAGCTGGCACCATCCGGCCATATCAAGGTTGACC GCGTCTCCCGCACCAACATCCCCGGTGTGTACGCAGCAGGTGACTGTACT GACCTATTCCCACTGGCGTCCGTTGCAGCGATGCAGGGCCGTATCGCCAT GTATCACGCACTCGGTGAAGGCGTGAGCCCCATCCGTTTGAAGACTGTTG CCACCGCAGTGTTTACCCGCCCAGAGATCGCAGCAGTAGGTATCACCCAT GCACAAGTTGATTCCGGCGAAGTGTCTGCTCGCGTGATTGTGCTTCCTTTG GCTACTAACCCACGCGCCAAGATGCGTTCCCTGCGCCACGGTTTTGTGAA GCTGTTCTGCCGCCGTAACTCTGGCCTGATCATCGGTGGTGTCGTGGTGG CACCGACCGCGTCTGAGCTGATCCTACCGATCGCTGTGGCAGTGACCAAC CGTCTGACAGTTGCTGATCTGGCTGATACCTTCGCGGTGTACCCATCATTG TCAGGTTCGATTACTGAAGCAGCACGTCAGCTGGTTCAACATGATGATCTA GGCTAA |
| 22 | GTGGGAACGGCGAGAGGGCTTTCTCAAGTTCTGAAAGAAGCCAGACAAGG TGGAAAACCCACTTCAGCCACCCAGAAAGTTCCACGCTCCGGCTACAGAT ATGACCTCGATGGGCTCCGCGGCATCGCGATCGCCTTCGTAGTTTTGTTC CATGTTTTCGTCGGAAAAGTCTCCGGCGGTGTGGATGTCTTCCTGCTGCT GTCTGGCTATTTCTTCTTAGGGTCGCAATTGCGTTATGCAGATCGTCCAGA TTCTTCCATCAACCCCTGGTGGCCGATTTGGCGCACGCTACGCAGATTACT TCCTGCGTTAGTGCTGGTGTTGGGCGTTTCCATGGTCCTCATCTTGGCGT GGGTGCCCAGACTGCAACGAATAGAAATAGCCAACCAAGCAGTAGCCAGC CTCTTCTATGTCCAAAACTGGGAGCTCGCATCCCAAGGTGCTGCCTACGG AGCAGCCTCTGCAGAAGTCAGTCCTTTCCAGCACTTGTGGTCCATGGCTG TGCAAGGGCAGTTCTACCTCTTTGCCATCTTGTTGAGCATGACGATCATCC TGATTCGTCGATACCGCCCCGAATACTCCGCAGTGCGACTAGCGACTCCT GTGCTGGCAGTGCTCACATCCGTATCATTTTTCAGTGCAATCCTGTGGCAT TTCATTGATCAATCAGTCAACTACTATTCCACCTTCACCAGGTTCTGGGAG CTCGGCCTTGGTGCACTATTGGTGCTGCATGCGCCTCGAATTTTGATTTCT GCGAAGACTAAATCAATACTCGCAGCCGTCGGTTTGTTTATGGTGCTATCC ACTGGATTCTTCATGGATGGCGCAGAGACTTTCCCTGGATTCCCCGCGCT GTATCCCATCTTGGGTGCTTGCTTAGTCATCCTTGGCGACGGTAAAATCTC GGTCTTTCTCTCCCGAAAATGGATGCTTTGGCTCGGCGATATCGCCTACCC GCTCTACTTGTGGCACTGGCCTCTGCTGATCATTTTCACCGCTTTGTTCAA CCAAGAAGAGCCATCCATCTGGCTGGGTATCGCCGTGATTATGCTGTCCC TTGGCTTGGCGCAGCTGACTAACAAATACGTAGAAAAACCAATGCAACAGC AGGGAAAGCGCCCACTATTTAGGGAATCCCGCAGTGCTGATGCTTTGCAA AAGCTGCGTTCCTCCAGGCCTGCCCGAATGCGTGCGTATGCGGGTATCGC AGTAGTCGCAGTCGGTTTCGCCCTGACATCTACTCCGCAGGTGCTCTACC AACGAGTCCTCGATACCCAAATGAGTGATCTCGATCCGGAGATCTACCCC GGTGCACTCTCCCTGGCGGGCTTCGATGCCCCCGAAGTAAAACCCAAACC AAACCCCTACGTGCTGGCCGATACCGTCTCACCCGCCTGGGCAAAAGGCT GCATGTCCGTCTTCGGCGACGATCCATCTGAACTAGCACCAGACAACTAC GACGAAGATTACTGCGCTTTCGGCAACCCCAACTCACCCGTCGAGGTCTA CCTCGTCGGCGGATCCCACGCCGAACAATGGATGGCACCACTCGACGCC CTAGGCAAGCAACACAACTTCAAAGTGATCCCCCTCGTCCGCCAATCCTG CCCCACCTTCGTGGTGGAACTCGACGGAATTTTCAGCGACGACTGCATCG AATTCAACGAAAAAGTGATCGAACGCCTCGCAGAAGTAAAACCCGACCTC GTGGTCTCCAACTCCACCAGACCGCTCCTTGAAAAAGGCCGCGGCATCGA CGAAGTGCCAGAATCATACATCACGCTGTGGGACTTCCTCGAAGCCGAAG ACATCCCATTCATGGGACTCCGCGACAACCCGTGGTTCATCCAGCCAGGA GGCGAAGGATGGATGGTGTCCCAATGCTACGAAGAAGAACAATCCTTGGT GGACTGCTCGATCACCAAAGACAACTTCTACGCACCAGTAGACCCCGCTG AAAAAGAACTAGCCATCAGAAACAATATGCTCGCAGTGGATACTTCACGAT GGTTCTGCCCAGACGACCTGTGTATCCCAGTGATCGGCAATGTGTATGTAT ACCGCGACGGCAACCACATGTCCGATGAATACGCACAATCTTTGGCTCCA TTCCTGTGGAGCAATATGCGCAGGCTTCTGGTCGCTTAG |
| 23 | ATGTCAACTGCATATTTTATTGCGCACGGAAACGATACGTACCGCCCAACG GAGCATTCCAGCGGTGCGTGGCGCGATGATGAACTCCACCTTGCACCGGT TGCCGGTTTGGTGATTCACCATATGGAACGTTGGCGCAGGGAAGTGGTTG GCGATGCTTTGGTGTTCAGCCGTTTTAGTTTGGAGGTCTTGGGCCAGATC GCTCGGGATGATGTCACCTTGAGAACAGAGATCGTTCGTCCCGGGCGCAC CATTGAACTTATTGAAACCGTGGCTGAAATCAACGGCAGAGTGACGATTCG TGCACGCGCCTGGTTGCTGAAGACCTCTGATCTTGCCCATATTTCTGGCGA TACTTTTGAAGCGTTGCCGTCAATGACTGAACTGGCAGATGTTAAGTCAGC GTTCCAGTGGTCTGGTGGATTTATGGACAGCATTGTGTTTGTTGAGGACGC TGAGAAAATTCCGGGCAAAAACCGCGCGTGGATTACCGGCGATGTGCAGT TGGTTGACGGCGAAGATTCTGAGCCTTTGGCAGAGTTTTCCAAATTCTTGG ATGTGGCCAATGGCGTGGCGGTTCGGGAAGATCCAAAGACTTGGATGTTC CCGAACGTGGATCTTACTTTCCATCTGTTCCGCCAGCCGGTGGGCACCAA GGTGGGTTTTGATACCCGCGCGGCGTTTGGACCCAACGGCATCGGTTTGA CCACGTCGGTGATTCACGATGCCGAAGGTCCTGTGGGCACGCTTAATCAA TCATTGACAGTGCGCCCAATTGCTGAGTTAAACAAGTGA |
| 24 | ATGAGTGCATTTGATTCGATCCTAAATGTTGAAGAGTGGATCAGTGACCAC TACCTCACCAATGACGATGCCAAAGGTGCCTCATTTTCCAAGCGGGTGCG CGAGCGCATTAAAGAATGGAAAACCACCGAGGACGCAACCCAGCAGAGTG GCCCTTTAACTCGTTTTTCCAGCAACCGCCTGCAGTTGCAGCATGCTCTTT CTGAGCTTGACGACGCCACCACCGCCGCCAGTTTAGTGGCCTCTGCACTG GGGTATGGTGTCCCCAGCGCGCGCCACGCGCAGCGCGGCTCCGACACAA TATCCTATTCCTCTTGGGTGGGAAATGCCGGCAGTGTGGAATTTCTTGCAG CGACTCCCGCTGAAAGCTTTGAAGAGAACTTCCGATCCCTTCCCCTTGAGC CAGTAGCGGTCAATGACAAGCCCCAGGATATCACCGCAGCCAAATTGGTG GGCCAGATTTTCCTTAGTGATACTCCCCCTGCTTTTGTTGTTATCACCGCT GGTAAATGGGTGGTTTTAGCCGAGCGTGAAACCTGGCCTCTAGGCCGCCA CCTAGCTATTGATATTTCCCTGGTGGTGGAACGTAATGACACCAAAGCCCA GGGTGAGATGCAGCAGACGGTCGTAGCACTAGCCCGCGAAAATACCGAG CGTGCCGCCGATGGCACCACCTGGTGGGAAGAAACCATTGAGCAATCCC GCGAACATGCTGTCAAGGTTTCTGGCGAGCTACGCAGTGCGGTGCGTGAA TCCATTGAAATCCTGGGCAATGACGTGCTCACACGCTATGAAGCTAAAGAG CTCTCCACCGCTGAGATCGACGGTGGCGAGCTAGCTAAGCAATCTTTGCG |
| | CTATCTCTACCGCATTTTGTTCCTGCTTTTTGCCGAGGCTTCACCAGAGCTT GAAATCCTGCCAACCGGCACCCCGGAATATGACGAGGGTTATGGCTTGGC ACGCCTGCGCGAGCTTATTCTTAATCCCCCAGTTACCCAGCATGCTCGCAC GGGCACCCACCTTTATGATTCCCTGCAGCTGCTGTTCACTCTGGTGGATAA AGGCCACCACCCAACAGATGCTAAGGCTGTAGCTTTTGATGCCGAGGCTG GAGAAGAAGGCCTGCACTTCCGCAACCTTTCAGCGGATCTCTTCCTCCCT GCAGCCACAGAACTTATTGATCGAGTTGGTCTTTCCAATGAAGCCCTAAAC AAGGTCTTGGAAAACCTCCTGCTCTCCCGGGTGCAATCCGGTAAAGACCG CGGCTTTATCTCCTATGCCACCTTGGGTGTTACCGAGCTTGGCCAAGTTTA TGAGGGTCTGATGTCCTATACCGGCTTTATCGCCCAGGAAGATCTTTTTGA GGTTGCACCACATGGCAAAGCCGATAAAGGTTCCTGGATGCTCCCGGTCT CAAAGGCTGATGAAGTCCCTGCCGATAGCTTTATCGAAGTTGATCAAGAAG CCCCTGGTGGCGGCGTAATCAAGGTGCGTAAACGCCACCCGCGCGGATC ATTTGTGTTCCGTCAGTCCTCTCGTGACCGCGAACGCTCAGCGTCCTTCTA CACCCCACAAGTACTCACCAGCTTTACTGTCACCCAGGCTATTGAAGAACT CCAGGCATCAAAGCGCATCACCACAGCCAATGATGTTCTCAGCCTCACCAT CTGTGAACCTGCCATGGGTTCCGGCGCCTTCGCTGTGGAAGCAGTACGCC AATTAGCAGAGCTTTATTTGGAATTGCGCCAAGAAGAACTAGAGCAGCAGA TTCCAGCGGAAGACCGTGCCAAGGAACTCCAAAAGGTCAAAGCGCACATT GCGCTGCACCAGGTTTATGGTGTGGACCTTAACAGCACTGCTGTGGAGTT GGCGGAAATCTCGCTGTGGCTAGACACCATGAATGCAGAAATGGACGCAC CTTGGTATGGCCTGCACCTGCGTAATGGTAACTCCCTCGTTGGTGCCACC CGTTCGCTGTATGCACCTAGTCTGCTTAATAAAAAAGCCTGGTTAACTGCT ACTCCAACCCGCTATCGGCTTGATGATATCGCGCAGGCTATTGATGAAAAC AAAGCAGAACCCCTCTTCAACCACGGCATCCACCACTTCCTCTTGCCCTCT ACTGGCTGGGGAGCCACTGCAGATGCCAAAGATCTTAAAGATCTTATGGC TACTGAAATCAAGGAGCTTAAATCTTGGCGTACTTCCATCCGTGCGTCTTT GAGTAAAACTCAGATTAAGCAGCTCAATAACCTTGCCCTACGCGTGGAAAC ACTATGGCGATTTGTGCTGATGCGTATTCGCATTGCAGAATCCCAGATCTC ACGTAGCACTACTCTCTGGGGTCAAGAGCCAGCTGAGGTTTCGGAGGTTG TCACACGTGAGCAAATTGAACAAGACCTCTTTGGCAATATTGATGGTGCAT ATAACCGTCTACGCTTGGTGATGGATGCTTGGTGTGCGCTGTGGTTCTGG CCTTTGGATGCTGTTGCTACCGCTGAGCATCCGGAGCGTCCAGCCCTTCC AGATCTTGATGAGTGGCTAGCCACCCTGACGGAGATTCTGGGTATTGATCT CCCTCTGAAGTCCAAAAACGAAAATCAGATTGTCTTAGGTCCAGATACCAA TTGGCTAGCCATTAATGATGCCGAGGCTACTGATCTTGGTTTTTCTGGGGC ATTGAGCTTTGAGCGTGTTAGCGCGAATCACCCGTGGATCAATGTTGCCC GCCAAGTGGCTAAACAACAGAGCTTCTTCCACTGGGATCTAGACTTCGCC CACGTTTTTGCCAAGGGTGGATTTGATCTGCAGGTTGGTAATCCACCATGG GTGCGACCAGATGTGAACTTTGAGGATCTGCTTGCTGAACATGATCCGTG GTGGGCTGTAATGAGTAAACCAACCCAGGCATCCAAAAAAGAACGCCAGA AGAATTTTCACAACAATCCTAAGAGCCTCGAACATGTGGTCAGTGGTGCAG GTGAAACCGTGGCTACTTCTGCGATCCTCGGTTCGGTTACTCTCTATCCGC ATCTTAAAGATCAACGCCCGGACCTCTACCGGGGCTTTATGGAAAAGACTT GGTCTAATGCCTCCCCGGCAGGTGCGATCTCACTGATCCACCCCGAATCC CACTTCACTGAGAAAAAAGCTGCCCCGCTACGTCGGGGAGCATATGAGCG TCTGCGCCGCCACTGGCAGTTCATTAATGAGTTGATTCTTTTTGACGTCCA CGACTTGGTTAAATATGGCGTACATGTCTATGGCGCTCCGCAGGAATCTAT |
| | TAACTTTTTAAGTGCTGCGTCGCTTTATCACCCACAAACAGTGCTTGATTCA TTTGATCATGACGGTTCAGGTAATCTCCCTGGTCTTAAAGACGACAATGGC AACTGGGACCGTCGCCCACACAAGGACCGTATCCAACTGGTCAATGCCGA TACTTTGACGGTGTGGAAGTCCATCCTGGAGGATGAACAAACGCCATACTT GGATACCCGCATGGTTTATACCGTCAACACGGAAGCAGCAGCAGTGTTGG AAAAGTTGGCTTCTGCACCTCGTATCAAAGAACTCGGGCTGCAGTTCTCCA GTGGCTGGAATGAAACCACCGATAAGAAAAAGGGATACTTTGACGTTGGTT GGGGCTACCCAGCTTCCTGGTCTGATGCCATTTTGCAGGGGCCGCACCTG GGTGTTGCTACACCAATGATCAAGCAGCCCAATCCGACAATGAAGCATAAT CAAGATTGGTCTGAAATTGATTTCGAGGCCATTCCTGCAAACTTCATACCT GCAACGGCGTACCAGCCCGATCGCCAAACAAAGCCCACTTATGATGCTGA CTACGGCACCTGGACTTTCGGGGACAAGCAGGTACCAGTTGCAGACACTT TCCGAATTGCATGGAGGGAGATGGCTGCCACCACGGGATTTAGGACTGTC TACCCATCAGTAATTCCACCGGGAGCCAACCATGTGCACACAGTTAATAGC GCTGCATCACGTTCAAACTTAAAAACCATTCTCGTTGGAGCACAGCTTGGT GCAATTCTAAGTGACTATTTTGCTCGGTCCTCGGGTTCAAGCCACATATTTA ACGACATTGTTCGCAAGATTCCACTTCCAAATTTCACATCCTTGGAAAAGCA GTTCGCCCGCACATACCTCCGCCTCAACTGCCTGACCTCAGCTTATGCCC CATTGTGGGAAGAGATCACCGGTGAGCCGTGGGATGTTCAGGTGCCTTTG CGCAATGCCGAGCAACGTCGAGCAGCGCAAAACGATATTGATGCCATGGT GGCATTGTCTTTGGGTATTAGTGCTGATGAGCTGTGCATGATTTATCGCAC TCAATTCCCAGTGATGCGTAGATATGATCAAGAAGATCATTTTGATGCCAAT GGCCGTAAAGTTCCTAAAGAGATCATCAAGCTGCAGCAGAAACTTAAAGAT GGCCAAGAGCTCAGCGTGGAAAAGCGCACCTGGGTGCATCCCCAATCAG AAGTGTCCTATACCTTTGAATATCCTTTCCGGGTGTTGGATCGTGAAGCTG ATCTGCGTGCTGCATATGCAAAATTTGAAAACCAGCTAAAGGAGCCATAG |
| 25 | ATGCGTAAAGGAATTTCCCGCGTCCTCTCGGTAGCGGTTGCTAGTTCTATT GGATTCGGTTCGGTGTTGTCTGGAACCGGCATCGCAGCAGCTCAAGACTC TGCATTTGACTACGGTATGGATCCGAGCATGAACTACAACCCGATCGACGA CATCAAGGATCGTCCCCAAGGGTTATCCAACCTTCCCTACTTCGGAAGCAA ACTAACCAGCTGGGGCTCATCAGATGCCACCGCCTCATCCGGCGTCGTGA CCTCCGCGCTCCCGCAGTACACCGATCCGCGCTACCCCCTCGGCAAAGA CGACCTGCCCAAGGCAACCATCGACATGGAGCCAGAAGTTCTTGCGCGCC TTGAGCGATTCGTCGGCGTTGACGGTGATCGCATCCGCCAAATCAACGCG TACTCGCCATCAATGGGACGCACCATTCCTCTAGTCTGGGTTGTTCCAGAA GACAACACCGTGCCTGGCCCAACGGTCTACGCACTCGGCGGCGGCGACG GTGGCCAAGGCGGCCAAAACTGGGTCACCCGCACCGACCTTGATGAGTT GACCAGTGAAAACAACATCAACCTCATCATGCCCATGCTCGGATCTTTTAG TTTCTACGCTGACTGGGCAGGCGAAAGCGAATCCATGGGTGGTGCGCAAC AGTGGGAAACATTCCTCATGCACGAACTACCCGAGCCGCTAGAAGCGGCC ATCGGCGCAGACGGGCAACGCAGCATCGTCGGCATGTCCATGTCCGGGG GATCGGTGCTGAACTTTGCGACGCATGACCCCAACTTTTATTCCTCTGTCG GCTCATTTTCTGGATGTGCCGAAACCAACTCCTGGATGGGGCGCCGTGGC ATCGCAGCCACTGCCTACAACGGCAATGTCGTGCCTGAGCAAATCTTTGG TGAAGTAGACAGTGATTACTCTCGCTATAACGATCCTTTGCTCAACGCTGC GAAGCTCGAAGAACAAGACAACCTCTACATCTTCGCCGGTTCCGGTGTGTT CTCTGAACTAGATGTCATCGGTGACAACGCACCGATTGATGAGGATGCGTT CAAAAACCGCGTCTTGGTTGGGTTTGAAATCGAAGCTATGTCCAACACCTG CACCCATAACCTCAAGGCTGCGACCGATCAAATGGGCATCGACAACATCA ACTACGATTTCCGACCAACCGGAACCCACGCCTGGGATTACTGGAACGAA GCGCTCCACCGCTTCTTCCCGTTGATGATGCAGGGCTTCGGCCTCGACGG TGGTCCCATCCCGATCTATAACCCTAACGGTGTGACCTCCAGCGAGTCTTC CTCAGAACTGTCTTCTGATGTGAGCCTTGGCACCGTGATCGGTAGTGTGG CGGGAAGCTCCGGATCAAGTGCAGGCAGCAGCGTCCGAGAGTTCATTGC AGGAAGTTCCGGATCAAGCCAATCAGCAGGAAGCTTCTACGAATAA |
| 26 | ATGACTACAACTACTTCTTCTGGGAAGTCTTCTGAAAAGATCAACCCCCTCT TCAAGCTCGGCAGTTTCCTAAGAAAAGGCACCGTCGGTTCTGAAGGCCAG CAGATTTTCCTTCAGGGCGGACGCCAAGCCGATGTGTTTTATCGCAACCG ATGGGCTTTCGATAAAGTCGTGCGCTCCACACATGGCGTGAACTGCACGG GCTCCTGCTCGTGGAAAGTGTATGTAAAAGACGGTGTGATCACCTGGGAA TCCCAGGCAGTGGATTACCCAACTACCGGTGCGGATATGCCCGACTATGA ACCACGTGGCTGCCCTCGTGGTGCATCATTTTCCTGGTACACCTACTCCCC AACCCGCATCCGCTACCCATACATCCGTGGCGTGCTAGTTGATATGTACC GCGAAGCCAAGGAACGCCTGGGCGATCCGGTGCTGGCGTGGCGCGACAT TGTAGAAACCCCAGAAAAGCGCAAAGCATATGTATCCCAGCGGGGCAAAG GTGGCCTCATCCGCGTTCAGTATGAGGAAGCCATGGAGATTGCTGCGGCA GCCCATGTGTACACCATCCGCCAATACGGCCCCGACCGCATTCATGGATT CACCGTTATTCCCGCAATGTCGCAGGTGTCTTACGGTGCTGGTACTCGCTT CTTGCAGATGATCGGCGGAGTGGCGCTGTCCTTCTACGATTGGTACGCCG ACCTCCCACCAGCATCACCACAAACTTTCGGCGATCAAACTGACGTTCCG GAATCTGGCGACTGGTACAACTCCAGCTACCTCATGATGTGGGGTTCCAA CATTCCGGTGACCCGCACGCCTGACTCCCACTTCATGGTGGAAGCCCGCT ACAAGGGCACCAAGGTTGTTGTGGTTTCCCCGGATTTCGCTGACTCCACC AAATTTGCTGATGAATGGGCACGCATCCACCCTGGTACTGACGGCGCACT CGCCTTTGCCATGGGCCATGTGATCTTGAAGGAATTCCATGTTGACAAGAA GACGCCGTACTTCATGGACTACATGCGCAAATACACGGACTCTCCTTTCCT CGTGGAATTAGATGAGCACGGCGATGGCACCTACACCCCAGGTAAATTCC TCACTGCAGACCGCGCAGCTGATATCTCCCCAGCGCTTGCCGCCACTCCA AATGCCACCCACCGTCTCCTTGTGCTGCAAAAAGATGGCTCAGTTGTAGAT CCCGGTGGCACTGTCGCGGACCGTTGGGGTGAAGAAGGCATGGGTAAGT GGAATCTGCGCTTAGACGGCGTAGATCCAGTGATGACTATTGCAGATGTA CAGACTGACACCGAAACTGCGGAAGTCCTCTTCCCCCGCTTCGATCTCCC AGCAACTGCCACCCAAGAAGGCCCCATTGGTGCTGGCACCATCAGCCGG GGCGTTCCCACCATCACGTTGAATGGCCGAAAGTACACCACTGTCTTTGAT GTGTTGCTCGCACACTACGGTGTGAACCGCGAAGAGCTCAACCTTCCTGG TGAGTGGCCTAAGGATTTCCAGGATCCAGTCATGGGTACTCCTGCGTGGC AGGAATAGCTCACGGGTGTTCCTGCTAATCAGGCGATTCGTTTGGGTCGG GAATTTGCTCAGAATGCTGATGATTCCAAGGGCCGTTCCCAGATCATCATG GGTGCTGGTGTGAACCACTACTTCCATGCGGATTCTATTTATCGCACATTC TTGGCGCTGACCTCTATGTGTGGCACCCAAGGTGTTAACGGTGGCGGTTG GGCTCACTACGTTGGTCAGGAGAAACTCCGTCCAATGAATGGTTGGGCAC AGTATGCCTTTGCTACAGACTGGCAGCGTCCACCACGTCAGATGATCACC ACTGGTTTCTACTACCTCACCACGGATCAGTGGAGGTATGACAACACTCGT GCTAATCGTCTGGCTTCCCCACTGGCTAATCGTGGCACCGTGGGTGACAA AATGACGGCGGATACCTTGGTGGAATCCATGAAACGTGGATGGATGCCGT CATTCCCGCAATTCAACCGCAATCCCCTCATCTTGAGCCAGGAGGCGGAA GAAAAGGGCGTGTCTGTTTCTGACCATATTGTTCAGCAGCTCACCGATGGT GACTTGCAGTTCGCCTGCGAGGATCCGGATGCACCGGAAAACTGGCCAC GCATTCTGCTTAACTGGCGCACAAACCTAATGGGCTCTTCAGCTAAGGGCA CGGAGTTTTTCTTGCGCCATATGTTGGGTGTGGATTCTGATGCATCTGCTG AAGAAAACGCGCCGGAGGATCGTCCAAGTTCCATTGTGTGGAGGGATGAG GCTCCGGAAGGAAAGCTCGATTTGATGCTGACCACGGATTTCCGCAACAC TTCCACCACCTTGGTCTCGGATATCGTGCTGCCGGCAGCCACCTGGTATG AGAAGCATGATTTGTCCACCACGGATATGCACCCCTTCATCCACTCGTTCA ATGCTGCGATCAACCCACCGTGGGAGACGCGTACTGACTGGGAGGTCTTC CACGATCTCACCAAAGAATTCTCCTCACAGGCAGCAACCTGGTTGGGCAC CCAAACCGATGTGATCACCGCACCGATTGCCCATGACTCCCCGGATGAGC TCAATATGCCTGGCGGTATCGTGCCAGATATTGATGAGGTCGGGCTGATC CCTGGCAAGACGATGGCCAAGATCATCCCGGTGGAACGTGATTACTCCAA GGTGTATGAAAAGTGGACACACTTGGGACCACTCACCGCCAAAGCGGGTA CCGGAACCCACGGCACTGCGTTTAACGTGACCAAGCAAACCGAGGAGCTG GCGCTGATCAACGGCACCTCCATCACGGAGTCTGCTGGTGAACGCCCCC GACTTGATTCCGCAGTCAAGGCCATTGAGATGATTCTGCACTTAAGTGGCG TGTCTAATGGTGAGGTTGCCGCCGAAGGTTTCAAGAATCAGGGTGCACGC GTGGGCACCGACATGATGGATCTGATTGCAGACCACGAAGGTACCCACAT CAATTGGGACATGGTCAAAGAACGTCCCGCCGAGGTGATCACCTCACCGG AGTGGACTGGTTCCAAGAAGGACGGACGTCGCTACACCGCGTTTTCCATC AACATTGAATACGACAAGCCGTGGCACACCCTGTCTGGTCGCATGCACTA CTACCTCGACCACGATTGGTTTATTGATTACGGCGAGCAGTTGCCAATCTT TAGGCCACCGTTGGACAAGATCCACATCAATGGTGAGGTCGGCCCTGGCC AGTCGGTCACAGGCACCGACGGCGAACCAGAAGTAACCGTGCGTTATCTG ACCACCCACAACAAGTGGTCGATTCACTCGCAGTACTACGACAATCTGCAT GTGCTTTCTATTTCTCGTGGCGGCCAGGTGATCTGGATGTCCAACAAGGAT GCAGAGAAACTCGGTATCGCTGACAACGATTGGATCGAGGCTTATAACCG CAACGGCGTTGTTTCTGCTCGTGCGATTGTCTCCCACCGCATTCCTGAAG GCACCGTGTTTATGAACCACGCGCAGGAACGCACCGCTGGCACCCCGCT GAACGAGAAGTCTGGCAGGCGCGGCGGAACTCACAACTCTCTTACTCGAA TCATGATTAAGCCGGTCCATGTTGCCGGTGGCTACGGCCACTTAACCTATG GCTTCAACTACATCGGCCCAACCGGAAATAACCGCGATGAGGTCACCAGA ATTCGTCGCCGCTCCCAGGAGGTGCAGTACTAA |
| 27 | ATGACTACAGCTCTTGAAGAGCAGAACGCACAGCAGGCAGCCACTGCCGG CCGTGTCGTGCGTGTCATTGGTGCGGTCGTCGACGTGGAGTTTCCCCGCG GCGAGCTGCCAGCACTGTACAACGCACTTACTGTAGAGGTAACCCTCGAA TCAGTTAAGAAGACCGTTGTTCTCGAGGTTGCTCAGCACCTCGGCGACAA CCTCATCCGCACCATCGCTATGGCACCAACCGACGGACTTGTCCGCGGTG CTGCTGTAACCGATACTGCACGCCCAATTTCCGTACCAGTGGGCGATGTT GTTAAGGGCCACGTATTCAACGCTTTGGGCGACTGCCTAGACGACGTTTC CCTGAACAACAACCCAGAGATCGAGCGTTGGGGCATCCACCGCGAGCCA CCATCATTCGATCAGCTTGAGGGTAAGACCGAGATCCTGGAAACAGGCAT CAAGGTTATCGACCTTCTCACCCCTTACGTTAAGGGTGGAAAGATCGGCCT CTTCGGTGGTGCAGGTGTGGGTAAGACCGTTCTTATCCAGGAAATGATCA CCCGTATTGCACGTGAGTTCTCCGGTACTTCCGTGTTCGCAGGTGTTGGT GAGCGTACCCGTGAGGGCACCGACCTCTTCCTCGAAATGGAAGAAATGGG CGTTCTCCAGGACACCGCCCTGGTGTTCGGTCAGATGGATGAGCCACCAG GAGTCCGTATGCGCGTGGCTCTGTCCGGCCTGACCATGGCGGAGTACTTC CGCGATGTTCAGAACCAGGACGTGCTGCTGTTCATCGACAACATCTTCCGT TTCACCCAGGCAGGTTCTGAGGTTTCCACCCTTCTGGGTCGTATGCCTTCC GCCGTGGGTTACCAGCCAACCCTGGCTGACGAGATGGGTGTTCTCCAGGA GCGCATTACCTCCACCAAGGGCCGTTCGATTACCTCTCTGCAGGCCGTTT ACGTTCCTGCCGATGACTACACCGACCCGGCTCCAGCGACCACCTTCGCT CACTTGGATGCAACCACCGAGCTTGACCGCTCCATTGCTTCCAAGGGTATT TACCCAGCAGTGAACCCACTGACCTCCACCTCTCGTATTCTCGAGCCAGC AATCGTTGGTGAGCGTCACTACGAGGTTTCTCAGCGTGTCATCGGCATTCT GCAGAAGAACAAGGAACTTCAGGACATCATCGCCATCCTTGGTATGGACG AGCTTTCTGAAGAGGACAAGATCACCGTTGCACGTGCGCGTCGCATCGAG CGCTTCCTGGGTCAGAACTTCTTCGTTGCAGAGAAGTTCACCGGTCTTCCT GGCTCCTACGTGCCACTGACCGACACCGTCGACGCTTTCGAGCGTATTTG CAACGGCGACTTCGACCACTACCCAGAGCAGGCTTTCAACGGCCTCGGTG GTTTGGACGATGTCGAAGCTGCATACAAGAAGCTGACCGGAAAGTAA |
| 28 | ATGACGCCTACCCAAAATGAGATCCACCCGAAACATAGCTACTCCCCCATC CGCAAGGACGGTCTCGAGGTCCCGGAGACCGAAATCCGCCTCGATGACT CGCCAAGCGGCCCCAACGAACCCTTCCGCATCTACCGCACCCGTGGCCC AGAAACCAACCCCAAGCAGGGACTTCCGCGGCTGCGCGAGTCATGGATCA CCGCCCGCGGCGACGTTGCCACCTATCAGGGGCGCGAGCGTTTGCTTAT CGACGACGGCCGCTCGGCAATGCGTCGAGGTCAAGCTTCGGCTGAGTGG AAAGGCCAAAAACCAGCTCCTTTGAAGGCGCTACCTGGCAAAAGAGTCAC CCAAATGGCCTATGCACGTGCTGGCGTGATTACTCGTGAAATGGAGTTTGT AGCGCTGCGCGAACACGTTGATGCGGAGTTTGTGCGCTCTGAGGTGGCG CGCGGTCGGGCCATTATTCCCAACAACGTCAACCACCCCGAATCTGAACC GATGATTATTGGTCGCAAATTTTTGACCAAAATCAACGCCAATATTGGCAAT TCTGCGGTCACCTCTTCAATCGAGGAAGAGGTGTCCAAGCTGCAGTGGGC CACGCGCTGGGGTGCCGATACCGTGATGGATCTATCCACCGGCGATGATA TTCACACCACCCGCGAATGGATTATCCGCAACTCCCCCGTTCCTATCGGCA CCGTCCCGATCTACCAAGCGCTGGAAAAAGTAAATGGCGTGGCCGCAGAC CTTAACTGGGAAGTATTCCGCGATACCATCATTGAGCAGTGTGAACAAGGC GTGGACTATATGACCATCCACGCCGGCGTCCTGCTGGCTTATATCCCACT GACTACCCGTCGTGTCACCGGCATTGTCTCCCGCGGCGGATCCATTATGG CCGGTTGGTGTCCGGCGCATCACCGCGAATCATTCCTCTACGAGCATTTC GACGAGCTGTGCGAAATCTTTGCACAATATGACGTCGCATTCTCCCTCGGT GATGGCCTACGCCCCGGATCGCTTGCCGATGCCAACGACGCCGCGCAAT TCGCCGAGCTGAAAACCATTGGTGAGCTCACCCAACGCGCCTGGGAATAC GATGTACAAGTAATGGTCGAAGGACCTGGACACGTGCCACTAAACATGAT CCAGGAAAACAACGAGCTGGAACAAAAGTGGGCAGCGGACGCACCTTTTT ACACTCTTGGACCACTAGTTACCGACATCGCTCCAGGTTATGACCACATCA CTTCTGCCATTGGTGCAGCTCACATCGCCATGGGTGGCACCGCCATGCTG TGTTATGTCACCCCGAAAGAACACCTTGGCCTGCCCAACCGTGACGACGT CAAAACCGGCGTAATCACCTACAAGCTCGCTGCCCACGCAGCAGATGTGG CCAAGGGTCATCCCGGCGCGCGTGCCTGGGACGACGCCATGAGTAAAGC GCGTTTTGAATTCCGTTGGAATGATCAGTTTGCGCTCTCCCTCGACCCCGA CACTGCAATCGCTTATCACGACGAAACCCTGCCGGCAGAGCCTGCGAAAA CCGCACACTTCTGTTCAATGTGTGGCCCGAAGTTCTGCTCCATGCGAATTA GCCAGGACATTCGCGATATGTTTGGCGATCAAATCGCGGAATTGGGGATG CCTGGGGTTGGGGATTCTTCTAGTGCTGTTGCTTCTAGTGGGGCACGGGA GGGGATGGCTGAGAAATCCCGGGAATTTATTGCTGGTGGTGCGGAGGTTT ATCGGCGTTAG |
| 29 | TTGCTTGCACGCTGTTTTGCTGCGGGCCGCAACGTGTCCACCCTGTGGCG TATCCTAGAATGCATGGCTTTTGCTGCTGAACATCCTGTCCTGTCCCACTC TGAGCACCGCCCGGTTGGTGAAATCGAGCGTAGCGATGACAAATTTGTTG TCGTTAGTGAATTTGAGCCTGCGGGTGACCAGCCTGCGGCTATTAAAGAG CTCGATGAGCGCTTGGATCGCGGTGAGCGGGACGTCGTTTTGATGGGTG CTACTGGTACGGGTAAGTCCGCGACTGCGGCGTGGTTGATCGAAAAGCAG CAGCGCCCCGCTTTGGTGATGGCGCCGAATAAGACGCTGGCTGCGCAGT TGGCTAATGAATTGCGGCAGCTGTTGCCCAATAACGCGGTGGAGTATTTC GTGTCTTATTACGATTACTACCAGCCAGAAGCGTATATCGCGCAGACTGAT ACCTATATTGAAAAGGACTCCTCGATTAATGAGGATGTGGAGCGTCTGCGT CACTCGGCGACGTCGTCTTTGCTGAGTAGGCGAGACGTCGTGGTTGTTAG TTCGGTGTCGTGTATTTATGGCTTGGGCACTCCACAGTCTTATCTTGACCG TTCCGTTGTGTTGAACGTGGGGGAGGAGATCGACCGCGATCGCTTTTTGC GCCTATTGGTAGATATTCAATACGAACGCAATGATGTGGGCTTTACTCGTG GTGCTTTCCGCGTGAAGGGCGATACCGTGGACATCATCCCGGCCTATGGG GAATTGGCGGTGCGCATTGAGTTTTTCGGTGATGAAATTGATGCGTTGTAC TACATCCATCCCCTGACTGGTGACACCATCCGGCAGGTGAATGAGATCCG TATTTTCCCAGCTACGCACTATGTTGCGGGACCTGAGCGGATGGAAAAGG CAGTCGCTGATATTAAGGCGGAGTTGGAAGTGCGCCTGGCTGATTTGGAG AACCGTGGCAAGTTATTGGAAGCGCAGCGTCTTAGGATGCGTACTGAATAT GACTTAGAAATGATCGAGCAGGTTGGTTTCTGTTCGGGCATTGAGAACTAT TCTCGCCACATTGATGGACGTGGGGAGGGAACCGCACCGGCCACGCTGA TTGACTATTTCCCAGAGGATTTCCTCACCATCATCGATGAGTCTCACGTGA CAGTCCCGCAGATCGGCGGCATGTTTGAGGGCGATATGTCCCGTAAACGT AACCTCGTAGAATTCGGTTTCCGCCTGCCATCCGCGATGGATAACCGCCC ATTGACCTGGGAGGAGTTCGATGAACGCCGTGGCCAAACGGTGTTCATGT CTGCAACTCCAGGCAAGTTTGAGATCGCTGCTGCTGATGGTGAGTTTGTG GAGCAGGTCATTCGCCCAACAGGTCTGGTGGATCCAAAGGTCACCGTCAA GCCAACGAAGGGGCAGATTGATGATCTGATCCATGAAATTCGCCAACGCA CCGATAAAGATGAGCGCGTTTTGGTCACCACATTGACCAAGAAAATGGCTG AGGATCTTACTGATTACCTGCTGGAAAACGGCATCCGCGTGCGCTACCTG CACTCAGATATTGATACCTTGCAGCGTGTGGAATTGCTGCGTCAGCTTCGC CTGGGCGAATACGATGTGTTGGTAGGTATTAACCTGCTGCGTGAGGGCCT TGACCTGCCAGAAGTCTCTCTGGTTGCGATTCTCGACGCCGACAAGGAAG GCTTCCTGCGCTCCACCACCTCACTGATTCAGACCATTGGCCGCGCCGCC CGAAATGTGTCCGGCGAGGTCATCATGTACGCCGACAAGATCACTGATTC GATGCAGTATGCCATCGAGGAAACCGATCGACGCCGTGAAAAGCAGGTCG CTTATAACAAGGAACACGGCATCGATCCGCAGCCGCTTCGAAAGAAAATC GCGGACATCCTCGACCAGGTCTATGACAATTCCGCTGATGGAGCAGGACC TTCTGCCTCTGGCGATGCGGCAGTCGTGGCTAAACCTGACGTGTCTAGCA TGCCCGCCAAAGAAGTGCAAAAGCTTATCGACGACCTCAGCGCTCAGATG GCTGCGGCCGCGCGGGAGCTCAAGTTCGAGCTGGCAGGGCGTCTGCGA GATGAGATCTTCGAGCTCAAGAAGGAACTGAGAGGTATCAAGGATGCCGG CATCTAA |
| 30 | ATGGGTGATTTGAAAATGCAACGTTCTGGAGGAGAACCCTTGGACACGAT CAAGGCCTATATTGCGCTAACGAAGCCCAGGGTTATTGAACTCCTCCTTGT CGCCACAATCCCCACAATGCTTCAGGCTGAACGCGGTGAGAACAACATTG TGCTCATCTTGCTGACTGTGTTCGGTGGCTGGATGGGTGCGGCCGCCGCC AACACCTTCAACATGGTGGCAGACTCCGATATTGATCAGCGCATGGGACG CACTAGGGCTCGCCCTTTGGTGCGCCACACCGTGAGTAATCGCGACGCCT CCATTTTTGCGTGGGTCCTGACAGTGGCCAGCTTCTTGTGGCTGTGGCTG CTGTGCGATTCGATGCTCGCCGGCATCTTCGTGTTGATCACGATTTTCTTC TACATTTTTGTCTACACCAAGTGGCTGAAGCGCCGCACGCACATGAATATC GTGTGGGGCGGAGCCGCAGGTTGTATGCCAGTGCTCGTCGGCTGGGCAG TGATCGTTGATCAGTTTGAGCCAGGCGTTCCACAGCAGTGGTGGCAGGCA ATTGTCCTGTTCATGGTGATTTTCTTCTGGACCCCACCTCACACCTGGGCT CTGGCCATGAAGTACCGCGAAGACTACAAGGCGGCTGGCGTCCCAATGCT TCCTGTCGTGCGCACCCCAGTCCAGGTCACCGCACAAATCGTGTGGTACT CCGTGGCAACTGTGCTGACCACCTTCTTGCTCATCCCAGCAACTGGTTGG ATCTACGCAGCGATCGCCGTCATTTCCGGCGTCACCTTCTTGTTCATGGCC ATCAAGCTGCACCTCGGCATCAAAAACGGTGGCAAGGTCAAGCCTCTGAA GCTGTTTATTTTGTCCAACAACTACTTGGCAGTCCTCTTCGTGGCATTGTCC GTCGACGCGGTCCTCGGCCTTGAGACCATCGGCGAGATGCTCGGCTGGA CCACCACCTTCTTCTAA |
| 31 | ATGAAGCTTTTCTCCCGCACCTCACTGGTTGCACTTGGTACCGCTGCAGCT ATCACTCTCTCCGGCGTAACCGCTCCTGCATTCGCTGATGAGGACTCCAA CGCAGCTGTAAGTGCTCTTAAGACCGCTGAAGATAATACCCCTGAGGCGC CAGGGGCTTCTACTCCCCTGAAGCTTGAGCAGCCTGGCACCATCACCGGC GTCCCTGGCAAGGCGATCACCCCGGTCACTGTTAAGGTCATCGCAGGCGA AGCAGAATCCTTCACCTCAGACAACCTTCCTTCTGGTCTCCTTATCGACAA CACCGGAAAGATCACCGGTACTCCAAAGAAGGAATTCACTGGTTCCGCAA AGATCATTGCCAAGAACGAAGCAGGCGTTGAAGCAGAAGTTTACGTTAACT TTGATTTCAACGAAGAGCCTTCCAGCGAAGAGCCTTCCAGCGGTTCTTCCG ACACTGACAACATCGAAAACTGGATCAAGATCATCACAGCAGTCATCGGTG CACTAACCACCATTTTAACCTTCAGTACCAAGCTGGATTCCTTCCTGAAGTA A |
| 32 | ATGCGCTCAGATGTCATCGAGTTACCGGAGGGGGTGAGCAAGGACGAAG CTGATCAGGTGGAGGTTGCGGAAGCGCGACTTAATGAGGGCGCACGATT GATGGCTGCAACTGGGTGTGAGGCTATGTGGCCAACAGGTTTCTCAGTTT GTGGCCGGATTCTTGACGCTTATCGGCAGGTTGGAGGTCAGTTGTCATGG CTTGGGCCACCGAAGTCAAACGAGTTGACCAATCCCGACGGTGTTGGCAA AAGAAGTGAATTTTTTGGTGGAGCCATCTATTGGCACCCAGACACAGGCG CTTATGCAGTGACCTTGGACGGTTTGCGACAGTGGGGGACCTTGAACTGG GAATCAGGGCCATTGGGGTACCCAACCTCTGGTCCGATGGATACAAACTA TCCCCTTACTCAGCGACAGACTTTTCAAGGTGGTGACAACTACTACAACCC ATTGACTGGCGGTGCTGTGTGGGGCGATATTAAACAGCGCTACGAAGAAC TTGGCGGCTCGAATCATGCCATTGGCATCCCGATCACTAATGAGCTACCTA GCGGTACTGAGTATTTTTACAATAATTTCTCCAATGGAACAATTTCGTGGCG AAATGATCGTCAGACACGGTTTATGTATTTGGCTACGCAGCGGGTGTGGG ATGCGTTGGGTCGGGAGACGGGTCGTTTAGGTTTTCCTGAAGCAGATGAA ACACCTGAGGTTTCTGGTCTATTCCATGTGGTGAATTTTGCGGAGCGCGG GGTGATTGCGTGGAATGGAATCCTAGGCGCCAGAGAGCTGTATGGTGATG TTTACTCCCTGTGGCTGCAATACCAAAATACCGATACTCCTTTAGGGTGGC CGATACCATCATTGACATCATTAAATGAGTCACTCGAACAAGAATTCACCA GAGGTGTTGTTTTAGGCTCAGGTGATGCACTGACATGGATTCCTGACGATG AAGAAAGAAGTTTGGAGGATTTCCTCCCAATTGGAAGTAGCGGCTCATCCT CATCGAGCCAAGAGATGACCCTGTTTTCCCAGCGTGCACAATACGTGGATT GCAAGAATCTTCCCGATTTAGATGAGCAGAGAAAAACTGAAAACAACATTG AAAAGAATGGTGGCCCGATCAAAAAAGAGTATAGTTCGCGAGGTTTCCCCA CCGAGTTCAGATTTGTGGTGAGAAAAGGGCATTATGACCGTTACAGGAATG AAGGCTGGGGATATTTAAAAAACTATTGCAAACACAACTTCGCCAACCACG CTATGGCTGAGGCCGTAGTAGATAAAGCGGTGATTGATTATGGCTCATCG CCAGGAACCAGCTATTACAAGTTCGAGAAAACGGTGTACTTTCTAGATTGC AGAACTTATACATTCAATAAGAACTCAGGATGTAAAGAAATGCACGCTCCG CAATGGGTGACTATTATTTACAATCCTCATACTTTCACTGGAGCAAATTCGA ACAGACCCAAGGGGGTAATTTCAGCATGGTGTAATTCAACCCCACCTGGT GGAATCGAACACGAGCCGGAAATTTCCCAATGTCCTGATCATGTGAATCTT TATAATAAGCTTCGCATATGA |
| 33 | ATGGTCAGCCAAACGGAAAGACAGCATGCAATTGCTTCTTTACTGGCACCA ACTGGTGCGGTGTCTGTAGGAGATTTAGCCGAGCATTTCCATGTGACAAC CGAAACAGTGCGACGTGATCTTCGGATCATGGAGTCACTGGGTTTGTTGC AACGAGTTCACGGTGGCGCCATTAGCCCAGAGCCCATGGGTACGTCTCCC CCTCGGCTGAAACCTGCCTTGGTTAAAGGAATGCCACCGGAACCCCGTGT TTTAGAACTTGCAGAAACTGCAGTTTCCCTCATCACACCTCTAGCACGCAG CATTTTCCTGGATTCAGGTTTAGCGTGCACGGCGATTGCCACGGTGTTGG GGGATCCTCCAGAAGATGCCAGGTGGACTGTTGTTACAAGTTCCCCCGGC GCTGTGATTGCCTTGTCCGCGACAGATGCCACCTCCACGGTGGTGCTGCA CGGGCAGGTTCACGGTAATTGTTCTTCAATCATTGGGTCCACGGCAGTAG ACATGATTTCGCAGTTGCGCGCTGATATCGCCTTCGTGGAGGTTGATGCG ATTCAATCCGATACAAGTCTGTGCACGTTTTTCCCGGAGACGATTCCCATC AAGCAAGCCATGATCAAAAACGCGGCTTTCACAGTTGCTGTTCTCAGCCCG AGATCTCCCCAAGATCAAGAACTTCAACTTTTGAAGCACCCTTTTTCCACCT TGGCTGATTTTGATGCCCTTGTTACCGATGACCACACGCTAGATTTTCCAG TTTTGCCCGACCACAACTTTCAGGTGGTAACCCCATGA |
| 34 | GTGACGCAGCAATTGAACCACGCAAAGGTAAATCAACATCCCGGTCAAGC CACCCTCCCAGAAACCGCAGAGGGGCAGGTTCGCACCTACGAGGTAAAAA CCTACGGCTGTCAGATGAATGTGCACGATTCTGAGCGCCTTTCGGGCCTG CTCGAGGAGGCTGGATACGTTGCTGCTCCGGAGGACACCACTCCGGATCT TGTCGTATTTAATACGTGCGCCGTGCGTGAAAACGCCGATATGCGCCTCTA TGGCACTTTGGGCAACCTGCGCAGCGTGAAAGAAAAGAACCCAGGCATGC AAATCGCTGTCGGTGGTTGTTTGGCTCAAAAAGACAAAGATACCGTGGTGA AAAAAGCACCGTGGGTGGACGTGGTGTTTGGTACCCACAACATTGGTTCC TTGCCAACCTTGCTTCAGCGCGCGGAGCACAATGCCCAAGCGGAAGTCGA AATTGTCGATTCCCTCGAGCAGTTCCCGTCAGTACTTCCTGCAAAGCGCGA GTCTGCTTACGCTGGTTGGGTGTCCGTATCAGTCGGATGTAACAACACCT GTACTTTCTGCATCGTTCCGTCGCTGCGCGGTAAAGAGCAGGACCGTCGA CCAGGAGACATCCTCGCAGAGGTACAAGCACTGGTGGATCAGGGAGTTAC CGAGGTAACTCTACTTGGCCAAAACGTAAATGCTTACGGCGTGAACTTTGT TGATCCTGAGCTAGAGCGCGATCGCAGTGCATTTTCCAAGCTGCTTCGTG CCTGTGGTGAGATCGAAGGCCTCGAGCGGGTTCGCTTCACCAGCCCTCAC CCTGCAGAATTCACCTCTGATGTCATTGACGCCATGGCAGAGACCCCAAA CATCTGCCCGCAGCTGCACATGCCACTGCAGTCCGGATCTGACAAGGTGC TCAAAGAGATGCGCCGTTCCTACCGATCCAAGAAGTTCCTCTCCATCTTGG ATGAGGTCCGTGCGAAGATCCCTCACGCCTCTATCACCACCGATATTATTG TCGGATTCCCTGGCGAAACAGAGGAGGATTTCCAAGCAACCCTCGACGTT GTCAAGAAGGCACGCTTTACTTCTGCTTACACCTTCCAATACAGCCCACGC CCTGGCACCCCTGCAGCGGAATATGAAAACCAGCTTCCAAAAGAAGTTGT GCAGGAACGCTACGAGCGCCTCATGGTCGTTCAGGAACAAGTCTGCGAAG AAGAAAACCAAAAGCTCATCGGCACCACCGTCGAATTGCTGGTCCAGGCT GGCGGAGGCCGCAAGAACGATGCCACCAAGCGCATGAGTGGTCGCGCAC GCGATGGACGCCTCGTGCACTTTGCGCCAGAGGGCGACATTGATGGTGA GATCCGCCCCGGCGATTTTGTCACTGTCACGGTGACTGAGGCCAAGCCTT TCTTCCTCATCGCAGACTCCGGTGTGCAGACCCACCGCCGCACCAAAGCT GGTGACAACTCTGCAGTTGGTCAAGTTCCAACCACAGCACCGATCGGTGT GGGCTTGGGACTGCCACAAATCGGCGCACCAAAGGTGGCTCCTGCCACA GAATCTGCCTGCTGCTCCATTAACTAA |
| 35 | GTGTCCAAAACAGAAGAAGGCCGTTCAGCGGCCATAATTATTTACGCGTTT CCAACTTTCATTCTGCTGGGCGCGATCATTGCGTTTATCTTCCCGGAACCA TTCATTCCGCTGACAAACTACATTAATATCTTCCTCACGATCATCATGTTCA CCATGGGTTTGACCTTGACGGTGCCCGATTTTCAGATGGTGCTTAAACGTC CACTGCCTATCTTGATCGGTGTAGTAGCGCAGTTTGTCATCATGCCATTCC TGGCGATCGTGGTTGCGAAAATGTTCAACCTCAACCCAGCACTCGCCGTT GGCCTTCTCATGCTGGGATCCGTTCCGGGTGGCACCTCCTCCAATGTGAT TGCGTTTCTCGCCCGAGGAGATGTCGCGCTATCGGTCACCATGACCTCTG TGTCCACCATTGTTTCCCCAATCATGACGCCTTTCCTCATGCTCATGCTGG CAGGTACTGAAACCGCCGTCGATGGTGGAGGCATGGCGTGGACTTTGGTA CAAACAGTGCTGCTGCCTGTGATCATCGGCCTAGTTCTGCGTGTCTTCTTG AACAAGTGGATCGACAAGATTTTGCCGATCCTTCCTTATCTCTCCATCCTC GGTATCGGTGGCGTGGTGTTCGGCGCAGTCGCAGCCAACGCGGAACGAC TCGTGTCTGTCGGACTCATCGTGTTCGTTGCAGTTATCGTGCACAACGTAC TTGGATACGTTGTGGGATACCTCACCGGCCGTGTATTCAAATTCCCAGAAG CAGCAAACCGCACCATGGCGATTGAAATCGGAACCCAATCCTCAGGCCTC GCATCGGGAATGGCAGGACGATTCTTCACCCCAGAAGCAGCCCTTCCAGG TGCTGTCGCTGCCTTGGTCCACAACATCACCGGCGCAGTTTATGTTGGGC TGGTACGAAACAGGCCTTTGACTAAGGCATCAAGGAAGAAGGAATCCGTC GCGGTTTCCAGCTAA |
| 36 | ATGTCATTTACAGCTTTTCAAACAGCCCTGCTCGTGTGGTTTAGAGCAAAT GCCCGCGATCTTGCGTGGCGTGATCCCAATACTTCAGCATGGGGAATTCT CCTTTCAGAGGTGATGAGCCAACAAACTCCCGTCGCGCGAGTCGAGCCGA TTTGGCGTGAGTGGATGGAAAAATGGCCCACTCCGGAAGATTTCGCGAAT GCGAGCACCGATGAGATTTTGCGGTCGTGGGGCAAGTTGGGCTATCCACG TAGGGCGCTGAGGTTGAAGGAATGTGCGGAGGTGATCGTCGAAAAGCATG CCGGCGAGGTGCCGGATACGGTGGAGGCGCTGCTCGCGTTGCCGGGGA TCGGTGATTACACGGCGCGCGCGGTCGCGGCGTTTCATTTTGGGCAGCG CGTGCCGGTGGTCGATACGAACGTGCGTCGCGTGTACCAGCGCGCGGTA GCCGGACGTTACCTTGCGGGGCCTGCGAAAAAGCAAGAGCTTATCGACGT CTCCCTTCTCCTTCCCAACACTCACGCCCCAGAATTCTCTGCCGCAATAAT GGAGTTGGGTGCTCTTATCTGCACGGCCACTTCCCCAAAGTGTGACACCT GCCCACTGCTTGACCAGTGTCAATGGCAAAAACTTGGCTGTCCCTCCCCG AGTGAAGAGGAGCTGGCTTCAGCGAAAAAGCGTGTGCAGAAATTTGTGGG AACCGACCGACAAGTCCGCGGCCTGATCATGGACGTGCTGCGCAATGCCA CCGCACCTGTGCCACTATCCGCGATTGATGTCGTGTGGCCTGACGATGCC CAACGCTCCCGGGCGCTGTTTTCGCTCATTGAGGACGGACTCGCGGAACA AGATGAGGCGGGTTATTTCCACCTGCCACGATAA |
| 37 | ATGGTGAACACGTTGAACTCTAAAACCGTGAATGTACCCCGTTTTGCCAGA GGCGTTGTTGCTGCAGCCACAGCGCTATTTTTTGGCGCTTTGGTAAGCCTC GCGCCTAGTGCGTTGGCGCAGGAACCACCTGCAGTTGAGGCCGGAGCGT CAGGTTCTTTGAGCAACCTGGGTGCCTGCATCGCTGATAAAGGCACTCTT GATGTCATCATCATGATCGATGAGACAGAATCCTTGATCCATGAAGCTCGT GACGGCGTGGTCAACGCCAATGAACCAGGCGCGGACGCACAGCACCACC GTGTTCCTGCAGCACAGAGCTTTGTGGATGAGCTTCTAGCCAAGCAAAGC GATGGTGATCTGAACACCCGCATCCGTGTTGCTGGTTTCGGCCAGACGTA CAAATCTGGTGCCACTGATCCAGACAATTACGGAGCATGGACGCAACTAG ATGCCTCCACCGTTGGCGGAGTACAAGATGAAATCTCCCGCTTCGCTGAC CGCACCCAGGAGCAGTACACCAACTACGCCTCCGCGATTGAGGGCGCTTA CCAGGACTTCACTAGGTCCGGCTCTGAGGACGCCTGCCGCATGCTGGTGA CCTTCACCGACGGCGCACTGACCGCTCAAGAAGGCGCCGATGTTGCAGAA GCAGCACTGTGCGCGCCGGGTGGCGTCACCGATCGACTGCGCAGTGCTG GCATCACCCACATCGGCATCGGTTTATCGGCACCTACCAACCCATCTGATT TCAGCCTGCTGCGCGGAACCACCGCAGGTGGCGGAACATGTGGTGTCGA ACCAGCTAACGGTGCATTCTTCCCAGCAGATAACGTGGGCGGACTTTTCG CAGCATTCCGTGAAGCCCTTGCGATTGGTGGTGAAACAATTGGTGAAACC CGAGCTGGTGATCCTTTCAGCTTCACCCTGGACAACTCGGTGAACTCTGT GCGTTTCACCGCGATCGCCAAGGATGATCTCGGCCCGAACGCCCACTTGG TGCTCACCGCACCCAACGGCGAAACGGTTGAGCTCAAAGATTCTGGAAGC AGCGTCGCCAACAGCACTGACGTGAGTTGGGAAGCCGAAAGCAGCCCAG TAAAAATGGCTGATGGTTCCCTCAACCTGCAGCAAGGTGGGGATTGGAAG GGTGTCTGGCAGATTCAGTTCCAAGGAATTGATCCTGCAGCGGTTGATGG ACGCGTATTCAACTCAGTGGAGATCCAGCCTGACCTCCAGCTTGTGTTCAG TGGCGGTGATTCGACGTCAGGTGCACTTAACCTTCGCGATGATCAGCAGC TGAATATGCAGCTGGTGGGCCGTGATGGTCAGCCACGCATCCTTGAGGGG TCCGCGCTGGTCGATCTTGGTTTCACCCGCGCAGATACCGGCGAGTTCGC CCCTTTGGCTCAAGGAATTGATATTTCTGGCGGCGAATTAAGCTTCCCGCT GGATACGATTTCGCAGCTCCCAGCCATCGGCACGGTGGAAGCGCGTACCA CGATCACCACCGCAGGCGTCGATGATCTCCCCGGCACCACGTTGAGCCCA ATTCTCAACACCACGCGCATCACCATCACTCAGCGCGATATGCCTCAGCTG CCAGCGTCGGTTCGCTTCACAGCGGATGAGGACGTTGTTACCGTAGACAT CCCCATCACCGGCCCCGGCAAGGTATGGATTGCACCGGGAACCCAGCTC AGCGGAGTGCTTCCAGACGGCGTGGACGGCATTGCAGCATCAAGTACTTT CGACAGCCCAGATAATGCTTTGGTGCTCGGACTGGATGAGCAGGGCACGA TTCCTGTTGAACTAACCGTGAGCGATCTTCGTGACGGACTGGTCAACGGC TCGATTCCACTCCAGATCTCCAACGCTGAGGGCGCCAATGAAACCAGCGT GGATCTGCCGACAGAAGGCACGTTGAGCGTTCCCATTAACGCCTCCACTT TCGCATTGGCATTCATTTTGGCCCTTGTGCTTTCCCTCCTGATTCCGCTGC TCATTTTGTATATTGTGCGTTTCCTCTCCGCAAAGGTTCCGTCCTCTGCGAT GAGTGGCGTGCGCATTCCAGTGGAATTCTCCGGTGAAGCTCTGCGCTATG CGGGCAGCACTATGCCTGATCTCGCATCGCAAACCACCGCCACCAAGCAG GTCGTTGTTCATGGAGACACCTTCAATGTGGAAGGCCACAAACTTAAAGTC CAGCGCTTCCAGCTGAACCCGATTGCGTCTCCTGCAGTGATCGTGCAGAC CGACCCGTCGATCAGCTTCGACGGCAAACAAAAGGGCACACAAGCTAAAC TCCCGCTGGCGGTCCAAGGCAGTTGGTTCCTCACTGCAAGCGGCGCTGA CCCTTCCAAGATGGAACTCATCGCCCTGACAAACCTGCCGCTCGAGCAGG GCCAAATCGACCGCATGATCGCAGGTATCACCAGCAAAGCCCCTGATAGG GCACGCGAACTACAAAAATTGCTTGACGACGCCGCGACCTCCCAGCCCGC AAAGGTTCCACCGCGCGCCCCAGCCGCGCAGGGCCACGTCGAAAAGCAA GCTCCTAGTTTTGGCACTGGTTCCGGTGGTGTTTTCGGCTCCAGCAATGGT GGAGGCTTTGGCTCCGGTAGCGGATCCAACGACACAAATGGTGGATTTGG TTCCAGCGGCGGCTTCGGCGCGATAA |
| 38 | ATGGCTGACCGCACACCGACCACCGCCACGCCCCCGGGGTGGGTGCTGG TCGTCGATGATGAACAACCCCTGGCTCAGATGGTGGCCTCCTACCTCATC CGGGCCGGCTTCGACACCCGCCAGGCGCACACCGGCACCCAGGCCGTG GACGAGGCCCGTCGCTTTTCCCCCGATGTTGTGGTGCTGGATCTGGGGCT GCCCGAACTCGACGGCCTGGAGGTGTGCCGACGGATCCGCACCTTCTCG GACTGCTACATCCTCATGCTCACCGCGCGTGGCAGCGAGGACGACAAGAT CAGCGGTTTGACCCTGGGGGCGGATGACTACATCACCAAACCTTTTAGCA TCCGGGAACTGGTGACCCGGGTGCATGCGGTGCTGCGCCGCCCGCGCAC CAGCACCACCCCACCGCAGGTGACCACCCCCTTGATCGTTGGTGACCTCA TCCTTGACCCCGTCGCCCATCAGGTGTGGGTGGGGGAGACGACCGTGGA GCTCACCCGCACGGAGTTCGAGCTGCTGGTTGCCCTGGCCCTGCGCCCC GGCCAGGTGCTGACCCGCCACGACCTGATCACCGAGGTCTGGGACACCA CCTGGGTCGGCGATGAACGCATCGTCGATGTCCACATCGGCAACTTGCGT CGCAAGCTCGGCACCGACACCCGGGGCCGGGGGTTTATCGACACCGTGC GTGGCGTGGGCTACCGGGTGGGGCAGCCATGA |

### Example 3: Construction of mutant genes-introduced L-isoleucine producing strains

### Example 3-1: Construction of recombinant vectors for introducing mutant genes

In order to confirm the effects of variants identified in Example 2, vectors capable of introducing them on chromosome were constructed.

Specifically, in order to introduce each of the NCgl0337, NCgI0658, NCgI0674, NCgI0686, NCgI0706, NCgI0885, NCgl1142, NCgI1165, NCgl1253, NCgl1315, NCgl1511, NCgl1588, NCgl1815, NCgl1859, NCgl1874, NCgI2245, NCgI2580, NCgI2629, and NCgI2863 gene mutations into KCCM12739P, vectors containing the target mutations were constructed as follows.

Genomic DNA of the KCCM12739P_mt4 strain was extracted using the G-spin Total DNA Extraction Mini Kit (Intron, Cat. No. 17045) according to the protocol provided with the kit, and PCR was performed using the genomic DNA as a template. The polymerase used was Solg^{™} Pfu-X DNA polymerase, and the PCR conditions were as follows: denaturation at 95°C for 4 minutes; followed by 27 cycles of denaturation at 95°C for 30 seconds, annealing at 60°C for 30 seconds, and extension at 72°C for 50 seconds; and a final extension at 72°C for 5 minutes.

The primer sequences used in the above experiment are shown in Table 5 below.

**[Table 5]**

| Primer | Sequence (5'→3') | SEQ ID NO |
|---|---|---|
| NCgI0337*_F | tgaattcgagctcggtacccTTTCGTGCGTTACGT ACTAA | 39 |
| NCgI0337*_R | gtcgactctagaggatccccCTATTGCTCATTGTT GGCCT | 40 |
| NCgI0658*_F | tgaattcgagctcggtacccACGGACCCAAGCAA GACCTC | 41 |
| NCgI0658*_R | gtcgactctagaggatccccAGATCGCACTCAAC GGTTTC | 42 |
| NCgI0674*_F | tgaattcgagctcggtacccTGCTGCTGTCTGGC TATTTC | 43 |
| NCgI0674*_R | gtcgactctagaggatccccCCAAGGATGACTAA GCAAGC | 44 |
| NCgI0686*_F | tgaattcgagctcggtacccGCCCACGCATTATTT CCGGA | 45 |
| NCgI0686*_R | gtcgactctagaggatccccCAGACCACTGGAAC GCTGAC | 46 |
| NCgI0706*_F | tgaattcgagctcggtacccGGTGCGGCCCCTGC AAAATG | 47 |
| NCgI0706*_R | gtcgactctagaggatccccGGCTACTTCTGCGA TCCTCG | 48 |
| NCgI0885*_F | tgaattcgagctcggtacccGGAAACTATGGGGA TTTTGG | 49 |
| NCgI0885*_R | gtcgactctagaggatccccGCGGCTCGGGTAG TTCGTGC | 50 |
| NCgl1142*_F | tgaattcgagctcggtacccTTAGCACGAGTGTT GTCATA | 51 |
| NCgI1142*_R | gtcgactctagaggatccccGGCACTGTCGCGG ACCGTTG | 52 |
| NCgI1165*_F | tgaattcgagctcggtacccGCTCCATTGCTTCCA AGGGT | 53 |
| NCgI1165*_R | gtcgactctagaggatccccTCATTCGCCCAGAC GGCGAA | 54 |
| NCgI1253*_F | tgaattcgagctcggtacccACCAATGGCAGAAG TGATGT | 55 |
| NCgI1253*_R | gtcgactctagaggatccccCAATCGAGGAAGAG GTGTCC | 56 |
| NCgI1315*_F | tgaattcgagctcggtacccGTGTCTTATTACGAT TACTA | 57 |
| NCgl1315*_R | gtcgactctagaggatccccCGTGGCCGGTGCG GTTCCCT | 58 |
| NCgI1511*_F | tgaattcgagctcggtacccCACAGCAGCCACAG CCACAA | 59 |
| NCgI1511*_R | gtcgactctagaggatccccATAGGCGATAGCAT GGCAAG | 60 |
| NCgI1588*_F | tgaattcgagctcggtacccTCACTTAAATAACAT CTTGC | 61 |
| NCgl1588*_R | gtcgactctagaggatccccTCACAAAGGATTAG TCGCGT | 62 |
| NCgI1815*_F | tgaattcgagctcggtacccATTTGCCCGGGAGG GCCTAA | 63 |
| NCgI1815*_R | gtcgactctagaggatccccTGATCGGGATGCCA ATGGCA | 64 |
| NCgl1859*_F | tgaattcgagctcggtacccCACTTTTAATAACTT CATAC | 65 |
| NCgI1859*_R | gtcgactctagaggatccccCAGACTTGTATCGG ATTGAA | 66 |
| NCgI1874*_F | tgaattcgagctcggtacccCAATGACATCAGAG GTGAAT | 67 |
| NCgI1874*_R | gtcgactctagaggatccccTTCGGGCCTGCTCG AGGAGG | 68 |
| NCgI2245*_F | tgaattcgagctcggtacccAATGCCCACGAAAC TGAAAA | 69 |
| NCgI2245*_R | gtcgactctagaggatccccGCTGGCAGGTACTG AAACCG | 70 |
| NCgI2580*_F | tgaattcgagctcggtacccCCCTTCTCCTTCCCA ACACT | 71 |
| NCgI2580*_R | gtcgactctagaggatccccCTTGGGCAGACAAC TACATT | 72 |
| NCgI2629*_F | tgaattcgagctcggtacccTGGAACATTCGGAA GGTTCG | 73 |
| NCgI2629*_R | gtcgactctagaggatccccCTCCTGCAGTGATC GTGCAGA | 74 |
| NCgI2863*_F | tgaattcgagctcggtacccCGGGCGGCGCAGC ACCGCAT | 75 |
| NCgI2863*_R | gtcgactctagaggatccccGAAATCAAAAACCC TGCTCA | 76 |

The obtained mutation-introduced fragments were cloned into the pDC24 vector (SEQ ID NO:115, Table 6), which had been digested with the restriction enzyme Smal, using the Gibson assembly method (D.G. Gibson et al., Nature Methods, Vol. 6, No. 5, May 2009; NEBuilder HiFi DNA Assembly Master Mix), thereby obtaining recombinant plasmids. Each vector containing the respective mutant insertion fragment was designated as PDC24-NCgl0337*, PDC24-NCgI0658*, PDC24-NCgl0674*, PDC24-NCgl0686*, PDC24-NCgl0706*, PDC24-NCgI0885*, PDC24-NCgl1142*, PDC24-NCgl1165*, PDC24-NCgl1253*, PDC24-NCgl1315*, PDC24-NCgI1511*, PDC24-NCgl1588*, PDC24-NCgl1815*, PDC24-NCgl1859*, PDC24-NCgl1874*, PDC24-NCgI2245*, PDC24-NCgI2580*, PDC24-NCgI2629*, and PDC24-NCgI2863*.

**[Table 6]**

| |
|---|
| The sequence of pDC24 (SEQ ID NO: 115) (5'→3') |
| GTTCGCTTGCTGTCCATAAAACCGCCCAGTCTAGCTATCGCCATGTAAGCCCACTG CAAGCTACCTGCTTTCTCTTTGCGCTTGCGTTTTCCCTTGTCCAGATAGCCCAGTA GCTGACATTCATCCGGGGTCAGCACCGTTTCTGCGGACTGGCTTTCTACGTGTTC CGCTTCCTTTAGCAGCCCTTGCGCCCTGAGTGCTTGCGGCAGCGTGAAGCTAGCT TTTATCGCCATTCGCCATTCAGGCTGCGCAACTGTTGGGAAGGGCGATCGGTGCG GGCCTCTTCGCTATTACGCCAGCTGGCGAAAGGGGGATGTGCTGCAAGGCGATTA AGTTGGGTAACGCCAGGGTTTTCCCAGTCACGACGTTGTAAAACGACGGCCAGTG AATTCGAGCTCGGTACCCGGGGATCCTCTAGAGTCGACCTGCAGGCATGCAAGCT TGGCGTAATCATGGTCATAGCTGTTTCCTGTGTGAAATTGTTATCCGCTCACAATTC CACACAACATACGAGCCGGAAGCATAAAGTGTAAAGCCTGGGGTGCCTAATGAGT GAGCTAACTCACATTAATTGCGTTGCGCTCACTGCCCGCTTTCCAGTCGGGAAACC TGTCGTGCCAGCTGCATTAATGAATCGGCCAACGCGCGGGGAGAGGCGGTTTGC GTATTGGGCGCTCTTCCGCTTCCTCGCTCACTGACTCGCTGCGCTCGGTCGTTCG GCTGCGGCGAGCGGTATCAGCTCACTCAAAGGCGGTAATACGGTTATCCACAGAA TCAGGGGATAACGCAGGAAAGAACATGTGAGCAAAAGGCCAGCAAAAGGCCAGG AACCGTAAAAAGGCCGCGTTGCTGGCGTTTTTCCATAGGCTCCGCCCCCCTGACG AGCATCACAAAAATCGACGCTCAAGTCAGAGGTGGCGAAACCCGACAGGACTATA AAGATACCAGGCGTTTCCCCCTGGAAGCTCCCTCGTGCGCTCTCCTGTTCCGACC CTGCCGCTTACCGGATACCTGTCCGCCTTTCTCCCTTCGGGAAGCGTGGCGCTTT CTCAATGCTCACGCTGTAGGTATCTCAGTTCGGTGTAGGTCGTTCGCTCCAAGCTG GGCTGTGTGCACGAACCCCCCGTTCAGCCCGACCGCTGCGCCTTATCCGGTAACT ATCGTCTTGAGTCCAACCCGGTAAGACACGACTTATCGCCACTGGCAGCAGCCAC TGGTAACAGGATTAGCAGAGCGAGGTATGTAGGCGGTGCTACAGAGTTCTTGAAG TGGTGGCCTAACTACGGCTACACTAGAAGGACAGTATTTGGTATCTGCGCTCTGCT GAAGCCAGTTACCTTCGGAAAAAGAGTTGGTAGCTCTTGATCCGGCAAACAAACCA CCGCTGGTAGCGGTGGTTTTTTTGTTTGCAAGCAGCAGATTACGCGCAGAAAAAA |
| GGATCTCAAGAAGATCCTTTGATCTTTTCTACGGGGTCTGACGCTCAGTGGAACGA AAACTCACGTTAAGGGATTTTGGTCATGAGATTATCAAAAAGGATCTTCACCTAGAT CCTTTTGGGGTGGGCGAAGAACTCCAGCATGAGATCCCCGCGCTGGAGGATCATC CAGCCCTGATAGAAACAGAAGCCACTGGAGCACCTCAAAAACACCATCATACACTA AATCAGTAAGTTGGCAGCATCACCCGACGCACTTTGCGCCGAATAAATACCTGTGA CGGAAGATCACTTCGCAGAATAAATAAATCCTGGTGTCCCTGTTGATACCGGGAAG CCCTGGGCCAACTTTTGGCGAAAATGAGACGTTGATCGGCACGTAAGAGGTTCCA ACTTTCACCATAATGAAATAAGATCACTACCGGGCGTATTTTTTGAGTTATCGAGAT TTTCAGGAGCTGATAGAAACAGAAGCCACTGGAGCACCTCAAAAACACCATCATAC ACTAAATCAGTAAGTTGGCAGCATCACCCGACGCACTTTGCGCCGAATAAATACCT GTGACGGAAGATCACTTCGCAGAATAAATAAATCCTGGTGTCCCTGTTGATACCGG GAAGCCCTGGGCCAACTTTTGGCGAAAATGAGACGTTGATCGGCACGTAAGAGGT TCCAACTTTCACCATAATGAAATAAGATCACTACCGGGCGTATTTTTTGAGTTATCG AGATTTTCAGGAGCTCTTTGGCATCGTCTCTCGCCTGTCCCCTCAGTTCAGTAATTT CCTGCATTTGCCTGTTTCCAGTCGGTAGATATTCCACAAAACAGCAGGGAAGCAGC GCTTTTCCGCTGCATAACCCTGCTTCGGGGTCATTATAGCGATTTTTTCGGTATATC CATCCTTTTTCGCACGATATACAGGATTTTGCCAAAGGGTTCGTGTAGACTTTCCTT GGTGTATCCAACGGCGTCAGCCGGGCAGGATAGGTGAAGTAGGCCCACCCGCGA GCGGGTGTTCCTTCTTCACTGTCCCTTATTCGCACCTGGCGGTGCTCAACGGGAA TCCTGCTCTGCGAGGCTGGCCGGCTACCGCCGGCGTAACAGATGAGGGCAAGCG GATGGCTGATGAAACCAAGCCAACCAGGAAGGGCAGCCCACCTATCAAGGTGTAC TGCCTTCCAGACGAACGAAGAGCGATTGAGGAAAAGGCGGCGGCGGCCGGCATG AGCCTGTCGGCCTACCTGCTGGCCGTCGGCCAGGGCTACAAAATCACGGGCGTC GTGGACTATGAGCACGTCCGCGAGGGCGTCCCGGAAAACGATTCCGAAGCCCAA CCTTTCATAGAAGGCGGCGGTGGAATCGAAATCTCGTGATGGCAGGTTGGGCGTC GCTTGGTCGGTCATTTCGAAAAAGGTTAGGAATACGGTTAGCCATTTGCCTGCTTT TATATAGTTCATATGGGATTCACCTTTATGTTGATAAGAAATAAAAGAAAATGCCAAT AGGATATCGGCATTTTCTTTTGCGTTTTTATTTGTTAACTGTTAATTGTCCTTGTTCA AGGATGCTGTCTTTGACAACAGATGTTTTCTTGCCTTTGATGTTCAGCAGGAAGCT CGGCGCAAACGTTGATTGTTTGTCTGCGTAGAATCCTCTGTTTGTCATATAGCTTGT AATCACGACATTGTTTCCTTTCGCTTGAGGTACAGCGAAGTGTGAGTAAGTAAAGG TTACATCGTTAGGCGGATCAAGATCCATTTTTAACACAAGGCCAGTTTTGTTCAGC GGCTTGTATGGGCCAGTTAAAGAATTAGAAACATAACCAAGCATGTAAATATCGTTA GACGTAATGCCGTCAATCGTCATTTTTGATCCGCGGGAGTCAGTGAACAGGTACCA TTTGCCGTTCATTTTAAAGACGTTCGCGCGTTCAATTTCATCTGTTACTGTGTTAGA TGCAATCAGCGGTTTCATCACTTTTTTCAGTGTGTAATCATCGTTTAGCTCAATCAT ACCGAGAGCGCCGTTTGCTAACTCAGCCGTGCGTTTTTTATCGCTTTGCAGAAGTT TTTGACTTTCTTGACGGAAGAATGATGTGCTTTTGCCATAGTATGCTTTGTTAAATA AAGATTCTTCGCCTTGGTAGCCATCTTCAGTTCCAGTGTTTGCTTCAAATACTAAGT ATTTGTGGCCTTTATCTTCTACGTAGTGAGGATCTCTCAGCGTATGGTTGTCGCCT GAGCTGTAGTTGCCTTCATCGATGAACTGCTGTACATTTTGATACGTTTTTCCGTCA CCGTCAAAGATTGATTTATAATCCTCTACACCGTTGATGTTCAAAGAGCTGTCTGAT GCTGATACGTTAACTTGTGCAGTTGTCAGTGTTTGTTTGCCGTAATGTTTACCGGA GAAATCAGTGTAGAATAAACGGATTTTTCCGTCAGATGTAAATGTGGCTGAACCTG ACCATTCTTGTGTTTGGTCTTTTAGGATAGAATCATTTGCATCGAATTTGTCGCTGT CTTTAAAGACGCGGCCAGCGTTTTTCCAGCTGTCAATAGAAGTTTCGCCGACTTTT TGATAGAACATGTAAATCGATGTGTCATCCGCATTTTTAGGATCTCCGGCTAATGCA AAGACGATGTGGTAGCCGTGATAGTTTGCGACAGTGCCGTCAGCGTTTTGTAATG |
| GCCAGCTGTCCCAAACGTCCAGGCCTTTTGCAGAAGAGATATTTTTAATTGTGGAC GAATCAAATTCAGAAACTTGATATTTTTCATTTTTTTGCTGTTCAGGGATTTGCAGCA TATCATGGCGTGTAATATGGGAAATGCCGTATGTTTCCTTATATGGCTTTTGGTTCG TTTCTTTCGCAAACGCTTGAGTTGCGCCTCCTGCCAGCAGTGCGGTAGTAAAGGTT AATACTGTTGCTTGTTTTGCAAACTTTTTGATGTTCATCGTTCATGTCTCCTTTTTTA TGTACTGTGTTAGCGGTCTGCTTCTTCCAGCCCTCCTGTTTGAAGATGGCAAGTTA GTTACGCACAATAAAAAAAGACCTAAAATATGTAAGGGGTGACGCCAAAGTATACA CTTTGCCCTTTACACATTTTAGGTCTTGCCTGCTTTATCAGTAACAAACCCGCGCGA TTTACTTTTCGACCTCATTCTATTAGACTCTCGTTTGGATTGCAACTGGTCTATTTTC CTCTTTTGTTTGATAGAAAATCATAAAAGGATTTGCAGACTACGGGCCTAAAGAACT AAAAAATCTATCTGTTTCTTTTCATTCTCTGTATTTTTTATAGTTTCTGTTGCATGGG CATAAAGTTGCCTTTTTAATCACAATTCAGAAAATATCATAATATCTCATTTCACTAA ATAATAGTGAACGGCAGGTATATGTGATGGGTTAAAAAGGATCACCCCAGAGTCCC GCTCAGAAGAACTCGTCAAGAAGGCGATAGAAGGCGATGCGCTGCGAATCGGGA GCGGCGATACCGTAAAGCACGAGGAAGCGGTCAGCCCATTCGCCGCCAAGCTCT TCAGCAATATCACGGGTAGCCAACGCTATGTCCTGATAGCGGTCCGCCACACCCA GCCGGCCACAGTCGATGAATCCAGAAAAGCGGCCATTTTCCACCATGATATTCGG CAAGCAGGCATCGCCATGGGTCACGACGAGATCCTCGCCGTCGGGCATCCGCGC CTTGAGCCTGGCGAACAGTTCGGCTGGCGCGAGCCCCTGATGCTCTTCGTCCAGA TCATCCTGATCGACAAGACCGGCTTCCATCCGAGTACGTGCTCGCTCGATGCGAT GTTTCGCTTGGTGGTCGAATGGGCAGGTAGCCGGATCAAGCGTATGCAGCCGCC GCATTGCATCAGCCATGATGGATACTTTCTCGGCAGGAGCAAGGTGAGATGACAG GAGATCCTGCCCCGGCACTTCGCCCAATAGCAGCCAGTCCCTTCCCGCTTCAGTG ACAACGTCGAGACAGCTGCGCAAGGAACGCCCGTCGTGGCCAGCCACGATAGCC GCGCTGCCTCGTCTTGGAGTTCATTCAGGGCACCGGACAGGTCGGTCTTGACAAA AAGAACCGGGCGCCCCTGCGCTGACAGCCGGAACACGGCGGCATCAGAGCAGCC GATTGTCTGTTGTGCCCAGTCATAGCCGAATAGCCTCTCCACCCAAGCGGCCGGA GAACCTGCGTGCAATCCATCTTGTTCAATCATGCGAAACGATCCTCATCCTGTCTC TTGATCAGATCTTGATCCCCTGCGCCATCAGATCCTTGGCGGCAAGAAAGCCATCC AGTTTACTTTGCAGGGCTTCCCAACCTTACCAGAGGGCGCCCCAGCTGGCAATTC CG |

### Example 3-2: Construction of mutant gene-introduced L-isoleucine production strains

The 19 vectors constructed in Example 3-1 were introduced into the isoleucine-producing strain KCCM12739P by electroporation, and strains in which the vectors were inserted into the chromosome through homologous recombination were selected on kanamycin-containing medium. Thereafter, the transformants in which the second recombination had been completed were subjected to PCR using the primer pairs shown in Table 7, and the strains into which the mutant genes had been introduced were confirmed.

**[Table 7]**

| Primer | Sequence (5'→3') | SEQ ID NO: |
|---|---|---|
| NCgI0337_F | CAATGCAGTGGGGGAGAG | 77 |
| NCgI0337_R | ACAGTGAAAACCTTACGG | 78 |
| NCgI0658_F | ATCAACGCCGGCTATGCGC | 79 |
| NCgl0658_R | CGTCTTCGGTGCGGGTGA | 80 |
| NCgl0674_F | ACACCAATGGGCTGTCAG | 81 |
| NCgI0674_R | ACTCGTTGGTAGAGCACC | 82 |
| NCgI0686_F | GCCGTTCGTATTCTCGGTG | 83 |
| NCgI0686_R | ACTCAGCAATTGGGCGCA | 84 |
| NCgI0706_F | TTGAACGTGATGCAGCGC | 85 |
| NCgI0706_R | TAGCCATTAATGATGCCG | 86 |
| NCgl0885_F | CCATCGCGCAACGGGGCG | 87 |
| NCgl0885_R | CACACCGGAACCGGCGAA | 88 |
| NCgl1142_F | CTTCAGCAGATGCATCAG | 89 |
| NCgl1142_R | TGACTCCACCAAATTTGC | 90 |
| NCgl1165_F | GCGCGTGGCTCTGTCCGG | 91 |
| NCgl1165_R | TTTTGAGTCCTTCCTGCG | 92 |
| NCgl1253_F | CCGCGATTTGATCGCCAA | 93 |
| NCgl1253_R | GCGCGAGTCATGGATCAC | 94 |
| NCgl1315_F | GAATGCATGGCTTTTGCT | 95 |
| NCgl1315_R | GATCAGATCATCAATCTG | 96 |
| NCgl1511_F | ATGAGCAAGAAGGTGGTC | 97 |
| NCgl1511_R | TAATTGGTTCAAATTTGG | 98 |
| NCgl1588_F | TACTATTTCTGCTGCTGG | 99 |
| NCgl1588_R | TCTTCTACGGTGTCAGCC | 100 |
| NCgl1815_F | GTACGTGGCGGGGGTAATAC | 101 |
| NCgl1815_R | CCCACACCCGCTGCGTAGCC | 102 |
| NCgl1859_F | CACGGTTTTCTTCGGCGA | 103 |
| NCgl1859_R | AGCGGTGACGGAATCAAG | 104 |
| NCgl1874_F | CCATGAGGCGCTCGTAGC | 105 |
| NCgl1874_R | AGTCTCCGATCCTGTCCG | 106 |
| NCgI2245_F | TTGGCTGCCTCAATAATC | 107 |
| NCgI2245_R | AATATCTTCCTCACGATC | 108 |
| NCgI2580_F | CCCGTCGCGCGAGTCGAG | 109 |
| NCgI2580_R | CAGCAGTGCGTCGCCTGC | 110 |
| NCgI2629_F | TTGGCTGCTCTTTAGAGG | 111 |
| NCgI2629_R | CTGCCGACAGAAGGCACG | 112 |
| NCgI2863_F | TAGCCCACGCCACGCACG | 113 |
| NCgI2863_R | GAAATCAAAAACCCTGCTCA | 114 |

The recombinant strains were designated as KCCM12739P△NCgI0337::NCgI0337*, KCCM12739P△NCgI0658::NcgI0658*, KCCM12739P△NCgI0674::NCgI0674, KCCM12739P△NCgI0686::NCgI0686*, KCCM12739P△NCgI0706::NCgI0706*, KCCM12739P△NCgI0885::NCgI0885*, KCCM12739P△NCgI1142::NCgI1142*, KCCM12739P△NCgI1165::NCgI1165*, KCCM12739P△NCgI1253::NCgI1253*, KCCM12739P△NCgI1315::NCgI1315*, KCCM12739P△NCgI1511::NCgI1511*, KCCM12739P△NCgI1588::NCgI1588*, KCCM12739P△ΔNCgl1815::NCgl1815*, KCCM12739P△NCgI1859::NCgI1859*, KCCM12739P△NCgI1874::NCgI1874*, KCCM12739P△NCgI2245::NCgI2245*, KCCM12739P△NCgI2580::NCgI2580*, KCCM12739P△NCgI2629::NCgI2629*, and KCCM12739P△NCgI2863::NCgI2863*, respectively.

### Example 4. Evaluation of isoleucine productivity of mutant gene-introduced L-isoleucine producing strains

In order to evaluate the L-isoleucine productivity of the strains constructed in Example 3-2, the strains were cultured as follows. The parental strain and the mutant strains were inoculated into 250-mL corner-baffled flasks containing 25 mL of isoleucine production medium, and cultured at 32°C for 60 hours with shaking at 200 rpm.

### <Production medium (pH 7.2)>

Glucose 10%, yeast extract 0.2%, ammonium sulfate 1.6%, potassium phosphate monobasic 0.1%, magnesium sulfate heptahydrate 0.1%, ferrous sulfate heptahydrate 10 mg/L, manganese sulfate monohydrate 10 mg/L, and biotin 200 µg/L (per 1 L of distilled water).

**[Table 8]**

| Strain | Concentration of L-isoleucine (g/L) |
|---|---|
| KCCM12739P | 2.1 |
| KCCM12739P△NCgI0337::NCgI0337* | 2.3 |
| KCCM12739P△NCgI0658::NcgI0658* | 3.1 |
| KCCM12739P△NCgI0674::NCgI0674* | 2.3 |
| KCCM12739P△NCgI0686::NCgI0686* | 2.4 |
| KCCM12739P△NCgI0706::NCgI0706* | 2.4 |
| KCCM12739P△NCgI0885::NCgI0885* | 2.2 |
| KCCM12739P△NCgI1142::NCgI1142* | 2.2 |
| KCCM12739P△NCgI1165::NCgI1165* | 3.0 |
| KCCM12739P△NCgI1253::NCgI1253* | 2.2 |
| KCCM12739P△NCgI1315::NCgI1315* | 2.3 |
| KCCM12739P△NCgI1511::NCgI1511* | 2.2 |
| KCCM12739P△NCgI1588::NCgI1588* | 2.2 |
| KCCM12739P△NCgI1815::NCgI1815* | 2.3 |
| KCCM12739P△NCgI11859::NCgI1859* | 3.1 |
| KCCM12739P△NCgI1874::NCgI1874* | 2.2 |
| KCCM12739P△NCgI2245::NCgI2245* | 2.5 |
| KCCM12739P△NCgI2580::NCgI2580* | 2.4 |
| KCCM12739P△NCgI2629::NCgI2629* | 2.4 |
| KCCM12739P△NCgI2863::NCgI2863* | 2.3 |

As a result, as shown in Table 8, it was demonstrated that most of the isoleucine-producing strains expressing the protein variants exhibited an isoleucine productivity equivalent to or superior to that of the parental strain. In particular, KCCM12739P△NCgI0658::NCgI0658*, KCCM12739P△NCgI1165::NCgI1165*, and KCCM12739P△NCgI1859::NCgI1859* showed a remarkable increase in isoleucine productivity compared with the parental strain KCCM12739P. These results indicate that more efficient production of L-isoleucine can be achieved by introducing: a protein variant in which the 27th amino acid of the protein (flavoprotein disulfide reductase) encoded by the NCgI0658 gene is substituted from glutamic acid (Glu, E) to aspartic acid (Asp, D); a protein variant in which the 461st amino acid of the protein (ATP synthase F0F1 subunit beta) encoded by the NCgl1165 gene is substituted from aspartic acid (Asp, D) to glutamic acid (Glu, E); or a protein variant in which the 75th amino acid of the protein (transcriptional regulator of sugar metabolism) encoded by the NCgl1859 gene is substituted from glycine (Gly, G) to valine (Val, V).

From the above description, those skilled in the art to which the present disclosure pertains will be able to understand that the present invention can be implemented in other specific forms without changing the technical spirit or essential features thereof. In this regard, the examples described above should be understood as illustrative and non-limiting in all respects. The scope of the present disclosure should be construed that all changed or modified forms derived from the meaning and scope of the claims to be described later rather than the above detailed description and equivalent concepts thereof are included in the scope of the present disclosure.

## Claims

1. A protein variant comprising an amino acid sequence in which the amino acid corresponding to the 27th position from the N-terminus in the amino acid sequence of SEQ ID NO:116 is substituted with aspartic acid, an amino acid sequence in which the amino acid corresponding to the 461st position from the N-terminus in the amino acid sequence of SEQ ID NO:117 is substituted with glutamic acid, or an amino acid sequence in which the amino acid corresponding to the 75th position from the N-terminus in the amino acid sequence of SEQ ID NO:118 is substituted with valine.

2. The protein variant according to claim 1, wherein the protein variant has, respectively, an amino acid sequence identity of 95% or more to less than 100% to the amino acid sequence of SEQ ID NO:116, SEQ ID NO:117, or SEQ ID NO:118.

3. The protein variant according to claim 1, wherein the protein variant comprises an amino acid sequence of SEQ ID NO: 2, SEQ ID NO: 8, or SEQ ID NO: 14.

4. A polynucleotide encoding the protein variant of claim 1.

5. A microorganism of the genus of *Corynebacterium* comprising at least one selected from the group consisting of the protein variant according to claim 1 and the polynucleotide encoding the protein variant.

6. The microorganism according to claim 5, wherein the microorganism of the genus of *Corynebacterium* is *Corynebacterium glutamicum.*

7. The microorganism according to claim 5, wherein the microorganism has increased L-isoleucine productivity, compared to a microorganism of the genus of *Corynebacterium* which does not comprise any one selected from the group consisting of the protein variant and the polynucleotide encoding the protein variant.

8. A method of producing L-isoleucine, comprising culturing the microorganism according to claim 5 in a medium.

9. The method of producing L-isoleucine according to claim 8, wherein the method further comprises recovering L-isoleucine from the cultured medium or microorganism.

10. A use for producing L-isoleucine, of the microorganism according to any one claim of claim 5 to claim 7.

11. A composition, method, product, process, or use **characterized by** at least one element disclosed in the present disclosure.
